(19) Europäisches Patentamt / European Patent Office / Office européen des brevets

(11) **EP 2 647 612 A1**

(12) **DEMANDE DE BREVET EUROPEEN**

(43) Date de publication:
**09.10.2013 Bulletin 2013/41**

(51) Int Cl.:
***C05D 1/00*** *(2006.01)*  ***C12N 11/00*** *(2006.01)*

(21) Numéro de dépôt: **13366001.9**

(22) Date de dépôt: **02.04.2013**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **03.04.2012 FR 1200991**

(71) Demandeur: **Polyor SARL**
**54000 Nancy (FR)**

(72) Inventeur: **Claude, Pierre-Philippe**
**54000 Nancy (FR)**

(54) **Utilisation de bactéries en application foliaire pour le renforcement azoté de la synergie nk**

(57) Utilisation d'engrais bactériens pour application foliaire contenant du potassium ($K_2O$), de l'azote (N), et des de bactéries stomatotaxiques et/ou stomatophiles, à pH compris entre 6,8 et 7,2, plus précisément entre 6,9 et 7,1 et avantageusement de 7 caractérisée en ce que ;
• les bactéries stomatotaxiques ont la faculté à se mouvoir à la surface de feuille (phylloplan) vers les cellules de gardes stomatiques (stomates),
• les bactéries stomatophiles ont elle la faculté à s'ancrer sur ou en proximité des cellules de gardes stomatiques (stomates),
et qu'elle s'effectue ;
• au moment ou l'indice de nutrition azoté (NNI) est égale ou supérieur à 1,00, et/ou
• au moment au moment où la teneur en nitrate du jus de base de tiges ou du jus pétiolaire qui représente un stock d'ions nitrate présente dans la plante est compris entre au moins 101 et 150%, plus particulièrement entre au moins 110 et 125%, voire plus avantageusement aux environs de 120% de la valeur seuil de déclenchement d'une apport complémentaire d'engrais azoté proposée, et/ou
• au moment où la teneur en nitrate de la sève xylèmique ascendante est comprise entre des valeurs d'au moins 6 et 14 moles/L, plus particulièrement entre des valeurs d'au moins 8 et 12 moles/L, voire avantageusement aux environs de 10 moles/L.

Figure 10

EP 2 647 612 A1

**Description**

**DOMAINE DE L'INVENTION**

L'agronomie et la physiologie végétale. Il est aussi question de microbiologie appliquée à la biofertilisation, notamment via l'application foliaire de préparations bactériennes et minérales.

**ÉTAT DE LA TECHNIQUE**

La synergie NK favorise l'intégration de l'azote dans le cycle de synthèse des protéines. Le renforcement de cette synergie NK est revendiquée commercialement (eg. CIGO-K Xpress™, 50 et 500 g/L de N et $K_2O$ par L, respectivement, de 3 à 4 L/ha, de 3 à 5 fois par campagne, Agronutrition SAS, 31390 France). En effet, le K est un cofacteur dans plusieurs enzymes impliquées dans le traitement post traduction (*post tranlational*) des chaînes peptidiques. Cette synergie s'explique aussi en partie par une relative richesse en N de la sève brute ascendante favorisant la photosynthèse et donc l'augmentation des besoins en $CO_2$ et $H_2O$ qui doivent transiter par les stomates ouvertes ; K étant le principale *osmoticum* responsable de la turgescence et l'ouverture des stomates, la synergie NK est vitre trouvée.

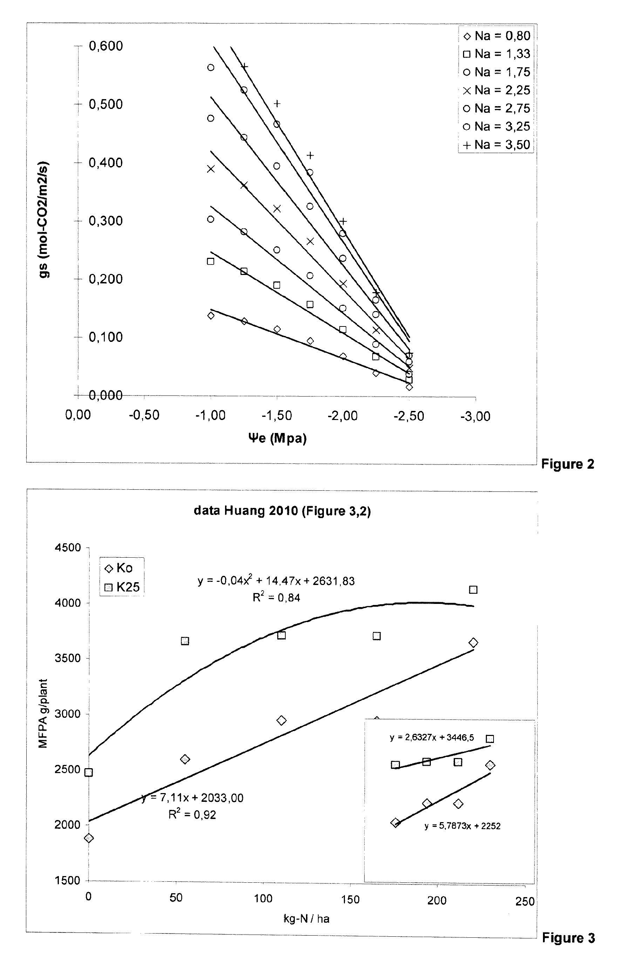

Or, cette synergie n'est pas maintenue à hauts taux de fertilisation azotée (Huang 2010, ici Figures 2 et 3 ; Dordas et Sioulas 2008, Wilkinson *et al.* 2007/2008, Fuentes et *al.* 2003, Carranca *et al.* 2009), ou plus généralement lorsque la teneur en N du feuillage (Na ; g- N / m²) est élevée en début de campagne céréalière dès la sortie d'hiver, ou plus généralement lorsque *l'indice de nutrition azoté* (NNI) est supérieur ou égale à 1, 00.

Cette dégradation de la synergie NK est due à deux mécanismes ;

1. une augmentation de N provoque une augmentation de NO (oxyde d'azote) régie par une augmentation, immédiate mais transitoire, des teneurs des sève en acide abscissique ([ABA] ;, en umol / m³ / sève) impliquée dans la fermeture des stomates ;

2. l'apport de $K^+$ provoque l'augmentation (négative) du *potentiel hydrique* des cellules épidermiques ($\Psi e$ ; MPa), ces cations devant par la suite migrer vers les cellules de gardes, faute de quoi cette augmentation de $\Psi e$ favorisera leurs fermetures.

Ce K contribue donc à l'ouverture des stomates qu'une fois activement transporté vers celles-ci. Or, les engrais foliaires K vont transitoirement augmenter négativement $\Psi e$, et sont donc *a priori* contre productifs en termes de *conductivité stomatique,* gs (mol-$CO_2$ / m² / s). Ils cesseront de l'être dès que les cations tels que $K^+$ diffuseront vers les cellules de gardes, cette rediffusion étant sensible aux [ABA]. Donc, et bien que N et K soient en principe synergiques, cette interaction est donc minée par la rétroaction des [ABA] sur gs, rétroaction fonction tant de Na que des concentration cellulaire de K et sont action sur $\Psi e$, et cela vraisemblablement en anticipation d'éventuels stresses hydriques et/ou oxydatifs à régimes photosynthétiques élevés.

Pire, la surproduction d'oxyde d'azote (NO) à partir des nitrates ($NO_3$) est parfois le fait de l'enzyme *nitrate réductase* (NR ; Shaner et Boyer 1976a, 1976b, Oliviera *et al.* 2010, Desikan *et al.* 2002) . Or, le NO, bien qu'éphémère, peut agir comme signal intercellulaire en conjonction avec l'ABA et favoriser la fermeture des stomates (Neill *et al.* 2003, Desikan *et al.* 2003, Crawford 2006) . Cette suractivité de la NR en présence de fortes teneurs en nitrates des sèves ascendantes explique en partie l'atténuation de la synergie NK à hauts taux de fertilisation azotée.

Donc, à la lumière du mode de fonctionnement osmotique des stomates, l'apport d'un engrais K foliaire va donc provoquer transitoirement une augmentation négative de $\Psi e$. En effet, cette diminution (augmentation négative) de $\Psi e$ va immédiatement favoriser la turgescence des cellules épidermiques et/ou para- stomatiques au dépend de celle des cellules de gardes, et donc provoquer la fermeture des stomates. Ce n'est que lorsque ce K aura repassé dans les cellules de gardes qu'il pourra provoquer la réouverture des stomates en augmentant $\Psi g$ (potentiel hydrique des cellules de gardes) . Or, le K ne repassera dans les cellules de gardes qu'activement que si [ABA] est jugulée (Dewar 2002, Wilkinson et Davies 1997, Borel et Simoneau 2002, Desikan *et al.* 2002, Neill *et al.* 2003) .

*Le rôle du pH*

Le passage des nitrates de la sève vers le *symplaste* (Wilkinson *et al.* 2007) implique un passage concomitant de protons ($H^+$) au dépend de l'*apoplaste.* La baisse conjointe du potentiel hydrique du symplaste en proximité des cellules de gardes suite à cette alcalinisation de l'apoplaste entraîne l'influx de K ; c'est ce K provoque la turgescences des cellules de gardes et l'ouverture stomatique. En contrepartie, cette alcalinisation de l'apoplaste, voire la suractivité de la NR en présence d'importantes concentrations de nitrates, provoqueront la fermeture des stomates ; d'où d'un point de vue agro-physiologique un certain équilibre. Par exemple, si la teneur en N des tissues et/ou de la sève ascendante via le xylème est supérieure à un certain seuil critique (Wilkinson et Davies 2008, Wilkinson *et al.* 2007 Radin et Parker 1976) les stomates réduiront leur aperture. L'alcalinisation des *apoplastes* en proximité des cellules de gardes et/ou de la sève xylémique permet en contribuant à l'activité de l'ABA amplifie ce signal anticipatoire de « détresse » (Wilkinson et *al.* 2007, Yamasaki 2000). *In contrario,* l'*acidification* de la sève xylémique et/ou des apoplastes mésophylliens, en réduisant [ABA], permets de maintenir la photosynthèse, la conductivité des stomates et/ou la croissance de foliaire. Il est donc tentant de chercher à simplement acidifier lesdits apoplastes si les taux de fertilisation N sont élevé.

Or, bien que cette acidification de l'ensemble de la surface de la feuille (phylloplan) soit loisible du fait que la production d'ABA est *in planta* la plupart du temps, elle agira négativement sur la physiologie enzymatique de la feuille (Kaiser et Spill 1991), voire nuire à chimie de la solution nutritive apportée conjointement. Par exemple et notamment, le pH optimal de l'enzyme *nitrate réductase* est proche de 7 (Dean et Harper 1988 *in* Neill *et al.* 2003, Kaiser et Brenelle-Behmsch 1991) ; l'acidification de l'ensemble du phylloplan et donc d'une partie de l'apoplaste réduira l'activité de la NR, et du coup annulera les bienfait de l'amplification de la synergie NK attribuable à une quelconque baisse d' [ABA] attribuable à cette acidification.

## DIVULGATION DE L'INVENTION

*Problème technique*

La synergie NK, pourtant favorable à l'augmentation du taux photosynthétique via un maintient de la *conductivité stomatique* (gs ; moles- $CO_2/m^2/sec$), ce dégrade si le taux de fertilisation azotée et/ou l'augmentation des teneurs en N du feuillage sont trop élevés. Une fois franchi un certain seuil pourtant encore loin de l'optimum agronomique, cette intensification de la fertilisation N devient moins, voire contre- productive en raison de cette dégradation de ladite synergie NK ; la baisse des rendements agronomique, protéique, voire unitaire (rendement agronomique / unité d'engrais N) en fonction de l'augmentation du taux de fertilisation N, même raisonnée, est inévitable.

Cette dégradation de la synergie NK est vraisemblablement le fait du jeu entre les concentrations *in planta* en NO et ABA favorisant l'efflux du K des cellules de grade stomatiques, et/ou l'augmentation des Ψe au dépend des Ψg (potentiel hydrique des cellules de gardes stomatiques) lors d'apports d'engrais K, apports pourtant essentiels à ladite synergie NK et au fonctionnement de plus de 60 enzymes, y comprises celles impliquées dans la protéogénèse. La solution technique devra réduire [ABA] en présence d'engrais foliaire NK, évitant ainsi une fermeture prématurée des stomates pouvant dégrader la synergie NK, et cela pus particulièrement en début de campagne céréalière dès la sortie d'hiver lorsque les valeurs de Na (g-N / $m^2$ de feuillage) sont élevées.

La dégradation de la synergie NK sera plus probable et problématique dès que l'NNI *(indice de nutrition azotée ;* Gastal et Lemaire 2002, Jeuffroy *et al.* 2002) sera égale ou supérieur à 1,00. En effet, ces teneurs en azote relativement élevées du feuillage, y compris et notamment suite à l'apport d'engrais foliaire NK contribuent aux augmentations de NO et/ou d'ABA, et donc à la fermeture immédiate et transitoire des stomates et donc à la baise de gs. Encore une fois, ces engrais K foliaires sont pourtant très importants à ce stade en raison de la forte activité enzymatique dédiée à la synthèse des protéines en plein montaison.

*Solution technique proposée*

Les apports d'engrais NK selon les préconisations agronomiques actuelles, au sol et/ou au feuillage, sont parfois contreproductifs en raison d'une dégradation de la synergie NK si l'un ou l'autre de ces apports élémentaires est renforcé de manière à dépasser NNI = 1,00. En effet ;

- l'accumulation histologique, *i.e.* en proximité des stomates (mésophylle), du K provoque une augmentation de Ψe (potentiel hydrique des cellules épidermiques) au dépend de Ψg (potentiel hydrique des cellules de gardes), et donc la fermeture des stomates ;
- l'accumulation histologique du N provoque lui une augmentation des teneurs en NO qui, en interaction avec l'ABA, va provoquer la fermeture des stomates.

Or, l'acidification de la sève xylémique et mésophyllienne peut en principe réduire la concentration d'ABA ([ABA] ) et augmenter Ψg au dépend de Ψe, contrant ainsi la susdite fermeture des stomates lors de tels apports d'engrais NK. De plus une telle réduction d' [ABA], de l'ordre de 15 à 40 umol- $ABA/m^3$ de sève xylémique et/ou mésophyllienne peut être effectuée via un baisse de pH de l'ordre de 0, 33 à 0, 67 unités commensurable avec le pouvoir acidifiant de bactéries. Il faut cependant que cette acidification relative soit *localisée* en proximité des cellules de gardes, faute de quoi l'activité de l'enzyme nitrate réductase (NR) sera affectée. En effet, le pH optimal de la NR étant proche de 7, l'acidification de l'ensemble de la surface de la feuille nuirait à l'activité de la NR obtempérant ainsi l'amplification de la synergie NK attribuable à un tel désamorçage d'ABA.

Concrètement, il s'agit donc d'une utilisation d'engrais bactériens pour application foliaire contenant du potassium ($K_2O$), de l'azote (N) et des de bactéries stomatotaxiques et/ou stomatophiles, à pH compris entre 6, 8 et 7, 2, plus précisément entre 6, 9 et 7, 1 et avantageusement de 7 caractérisée en ce que ;

- les bactéries stomatotaxiques ont la faculté à se mouvoir à la surface de feuille (phylloplan) vers les cellules de gardes stomatiques (stomates),
- les bactéries stomatophiles ont elle la faculté à s'ancrer sur ou en proximité des cellules de gardes stomatiques (stomates),

et qu'elle s'effectue ;

- au moment ou l'indice de nutrition azoté (NNI) est égale ou supérieur à 1,00, et/ou
- au moment au moment où la teneur en nitrate du jus de base de tiges ou du jus pétiolaire qui représente un stock d'ions nitrate présente dans la plante est compris entre au moins 101 et 150%, plus particulièrement entre au moins 110 et 125%, voire plus avantageusement aux environs de 120% de la valeur seuil de déclenchement d'une apport complémentaire d'engrais azoté proposée, et/ou
- au moment où la teneur en nitrate de la sève xylèmique ascendante est comprise entre des valeurs d'au moins 6 et 14 moles/L, plus particulièrement entre des valeurs d'au moins 8 et 12 moles/L, voire avantageusement aux environs de 10 moles/L.

L'engrais bactérien est appliqué sur le feuillage de la canopée de cultures, plus particulièrement agronomiques, et avantageusement des grandes cultures d'hivers choisies parmi un group comprenant les céréales d'hivers telles que le blé et l'orge, ou encore le colza.

L'engrais bactérien est ainsi appliqué sur le feuillage de la canopée entre ou aux stades BBCH 33 et 65, plus particulièrement BBCH 35 et 61 et avantageusement BBCH 39 et 59. Le feuillage de la canopée est donc avantageusement celui d'une culture céréalière d'hiver ayant produite entre 5 et 12 tonnes de matières sèches des parties aériennes (MSPA) par hectare entre ou à la mi - montaison et le début de la floraison.

Le potassium ainsi apporté au feuillage de la canopée représente entre 5 et 20%, et plus avantageusement environ 10% du potassium mobilisé par ce feuillage au moment de l'application dudit potassium. De plus, le ratio des teneurs en N et en K de l'engrais est compris entre 0,10 et 3,00. Plus précisément, le ratio des teneurs en N et en K de l'engrais est soit ;

- faible, soit de l'ordre de 0,10 et 1,00, si NNI important est proche mais inférieur à un maximum physiologique de l'ordre 1,25 à 1,30 (Jeuffroy *et al.* 2002), soit
- fort, soit de l'ordre de 1 à 3, si NNI est proche mais néanmoins supérieur à 1,00.

Divers engrais NK déjà commercialisés peuvent servir, y compris et notamment ici ceux de d'Agronutrition SAS, 31390 Carbonne, France). Par exemple, dans le cas d'NNI importants, des engrais NK à ratios N:K faibles tel que Cigo-K Xpress™ (50 et 500 g/L de N et $K_2O$, respectivement) peuvent être utilisés. *In contrario,* dans le cas d'NNI proches mais néanmoins supérieurs à 1,00, des engrais NK à ratios N:K forts tel que Fertigofol™ 313 (105 et 83 g/L de N et $K_2O$, respectivement) peuvent être utilisés.

La dose d'engrais azotés calculée et préalablement appliquée au sol, dite dose "X", représente au moins 101 et 125%, plus particulièrement au moins 105 et 115%, voire plus avantageusement aux environs de 110% de la dose X (kg-N/ha) autrement préconisée.

En ce sens, la somme des fournitures du sol en azote minéral (Nm ; kg-N/ha) plus les apports d'azote fertilisant au sol impliquée dans le calcul de la dose "X" (Justes *et al.* 1997), autrement préconisée est comprise entre au moins 101 et 133%, plus particulièrement entre au moins 110 et 125%, voire plus avantageusement aux environs de 112% de 250 kg-Nm/ha pour la culture des céréales à pailles et de 320 kg-Nm/ha pour la culture du maïs-grain (*cf.* Fuentes *et al.* 2003). Pareillement, la teneur ponctuelle des reliquats d'azote minéral des sols au moment de la récolte et impliquée dans le calcul de la dose d'engrais azotés, dite dose "X", est de l'ordre d'au moins 20 à 50 mg-N / kg-sol sec (*cf.* Carranca *et al.* 2009).

## BREVE DESCRIPTION DES DESSINS ET FIGURES

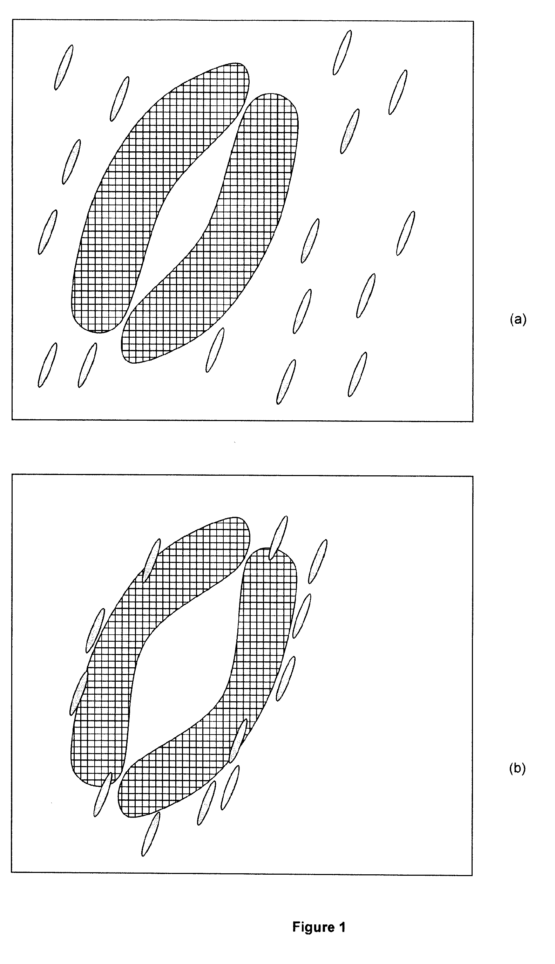

**Figure 1 :** Disposition dans le phylloplan de (a) bactéries non stomato- philes /- taxiques, et (b) de bactéries qui le sont puisqu'elles ont migrées ou sont « ancrées » sur ou en proximité des cellules de gardes (croissant hachurés) .

**Figure 2 :** Illustration de la dégradation de la synergie NK à partir des données agronomiques de Huang 2010 (figure 3.2) . La progression de la matière fraîche des parties aérienne (MFPA ; g/ plant de chou- fleur, *Brassica oleracea* var. *botrytis)* en fonction de l'augmentation des apports d'engrais N (kg- N/ha) n'est plus linéaire en présence d'engrais K (K25 = 25 kg- $K_2O$/ha) . En abîme, les mêmes chiffres, mais sans N = 0 kg- N / ha de manière à illustrer cette fois- ci cette même progression de la MFPA, bien qu'ici linéaire, toujours nettement moins rapide en présence de d'engrais K.)

**Figure 3 :** Illustration de la dégradation de la synergie NK (données Huang 2010, figure 3.6) telle qu'affectée par l'augmentation de l'apport de N et/ou de K. Même lors d'augmentations relativement faibles des taux de fertilisation N, ici et par exemple de 0 à 55 kg- N / ha, le taux d'augmentation de la MSPA (matière sèche des parties aérienne ; g/ plant de chou- fleur, *Brassica oleracea* var. *botrytis)* décroît en présence d'engrais K pourtant nécessaires à cette synergie. *Idem* si ce taux de fertilisation N passe de 55 à 220 kg- N / ha ; à de tels taux de fertilisation N, l'engrais K peut même avoir un effet délétère sur l'accumulation de la MSPA.

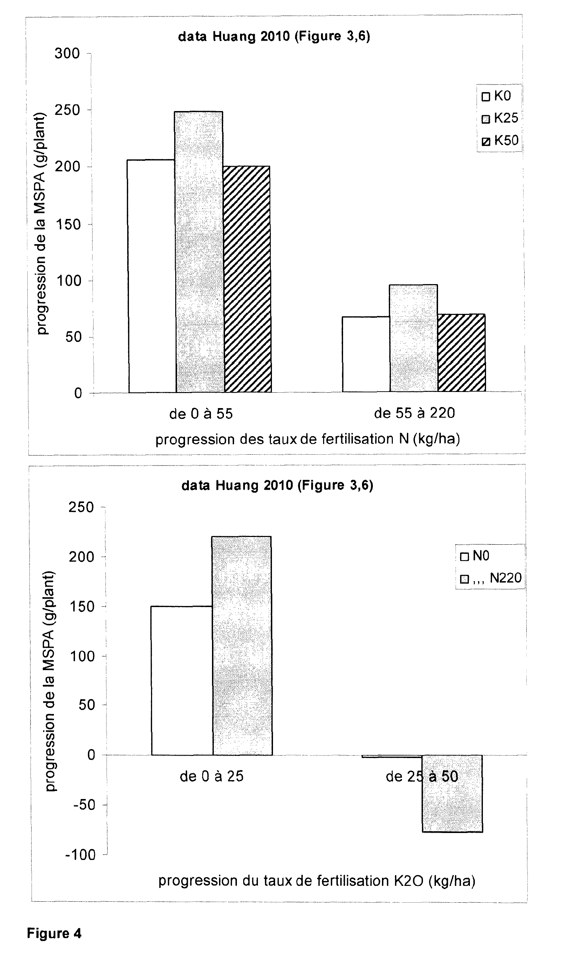

**Figure 4 :** Modélisation (selon Dewar 2002 et Müller *et al.* 2005, 2008) de la baisse de la conductivité stomatique (gs ; mol $CO_2$ / m$^2$ / s) en fonction d'une augmentation du potentiel hydrique des cellules foliaires épi- dermiques ($\Psi_e$ ; MPa) attribuable à l'application foliaire d'engrais K, et cela pour une gamme de teneurs en azote des feuillages, ($N_a$) de 0, 80 à 3, 50 g- N / m$^2$ de feuillage ; cette diminution progressive de gs est d'autant plus prononcée à hautes teneurs en N.

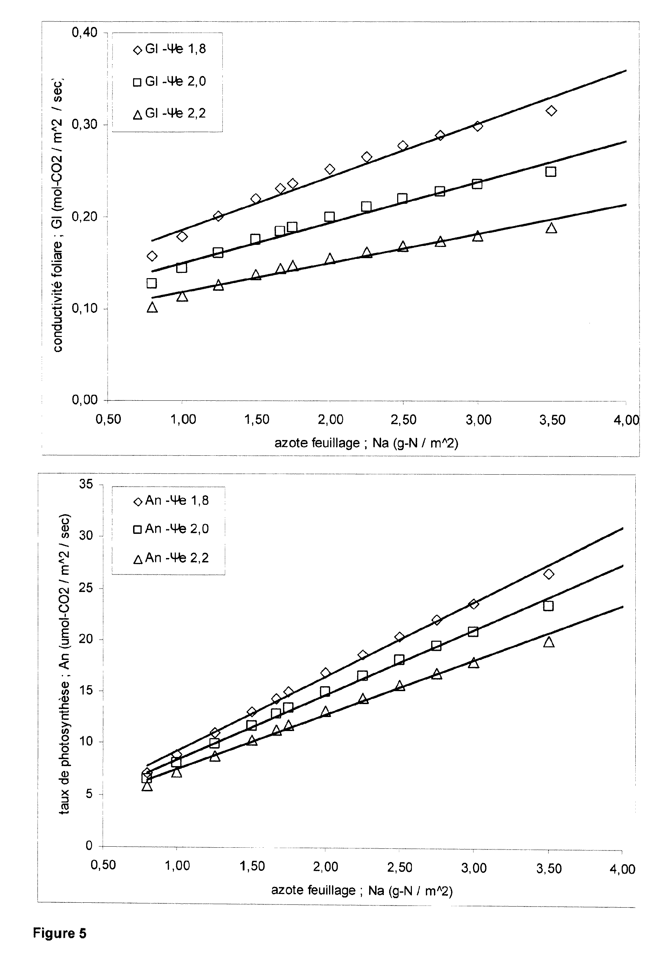

**Figure 5 :** Modélisation LEAFC3N de la conductivité stomatique au niveau foliaire (GI ; mol- $CO_2$/m$^2$/sec) et du taux (net) de photosynthèse (An, *alias* taux d'échange (foliaire) net du $CO_2$ ; umol/m$^2$/sec) en fonction de la teneur superficielle de feuillage en azote (Na ; g- N/m$^2$) pour trois valeurs de $\Psi_e$ (potentiel hydrique des cellules épidermiques, *alias parastomatiques* ; MPa) . L'apport au feuillage- 5 kg de $K_2O$ aux stades BBCH 37 à 59 (eg. blé tendre d'hiver) équivalent à ~ 10% du potassium histologique mobilisé à ce stade par les feuilles de la canopée provoque une augmentation négative de $\Psi_e$ de- 1, 8 à- 2, 0 MPa. Pour réduire $\Psi_e$ de- 2, 0 à- 2, 2 MPa il faut surdoser en plus d'éventuels engrais PK.

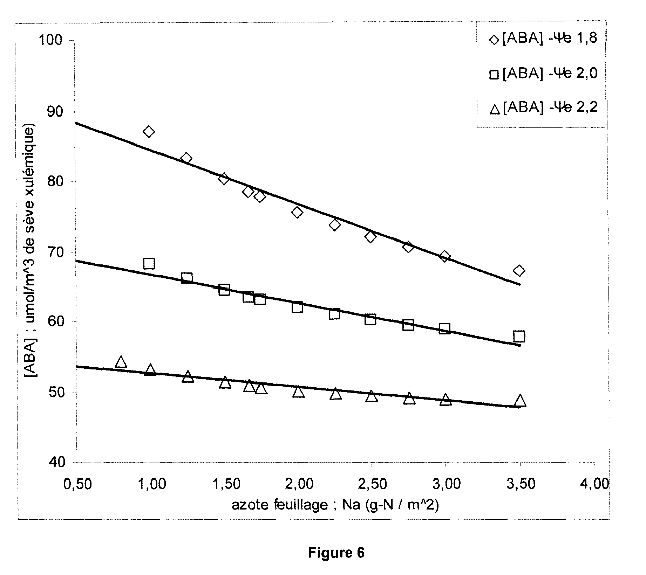

**Figure 6 :** Modélisation LEAFC3N- Dewar 2002 des teneurs en ABA des sèves xylémique ([ABA ; umol/m$^3$) selon l'augmentation des teneur en azote du feuillage (Na ; g- N/m$^2$ de feuillage) . En présence de potentiels hydriques épidermiques ($\Psi$e ; MPa) de plus en plus négatifs de- 1, 8 à- 2, 0 à- 2, 2 MPa suite à des apports d'engrais K foliaires et/ou au sol, [ABA] devra être réduite si GI et/ou An sont maintenus, sans pour autant varier drastiquement en fonction de Na. L'augmentation négative de $\Psi_e$ de 10% de- 1, 8 à- 2, 0 MPa est effectuée par une apport d'environs 4 à 5 kg- $K_2O$ par hectare au feuillage (canopée), par exemple ici d'une blé tendre d'hiver (BTH) entre les stades BBCH 29 et 55 ; la seconde augmentation à- 2, 2 MPa, soit ~ 20%, implique une surdose d'engrais PK au sol.

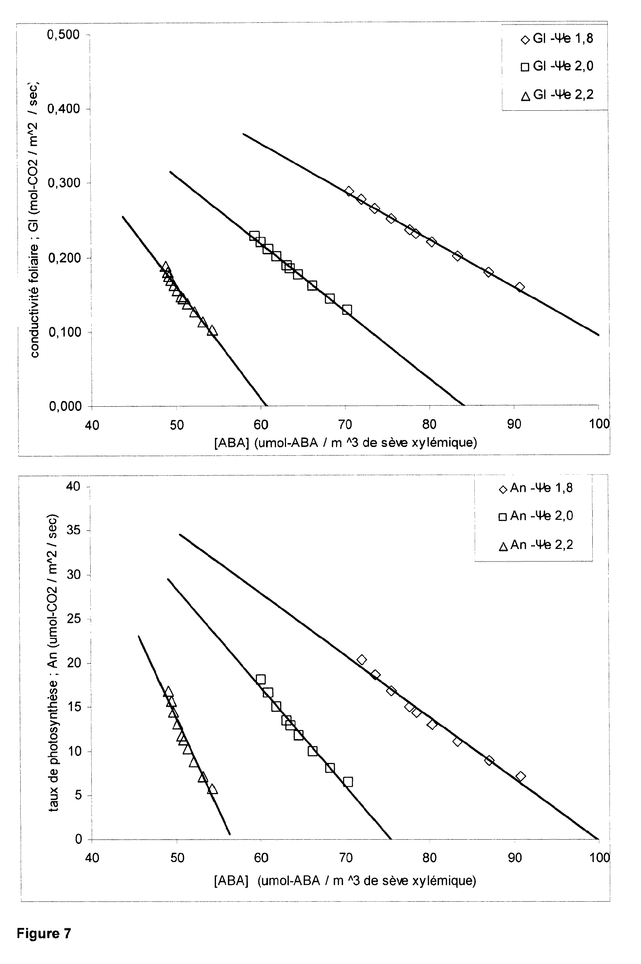

**Figure 7 :** Modélisation LEAFC3N- Dewar 2002 de la conductivité stomatique au niveau foliaire (GI ; mol- $CO_2$/m$^2$/sec) et du taux net de photosynthèse (An ; umol- $CO_2$/m$^2$/sec) selon les teneurs en ABA des sèves xylémique ([ABA] ; umol/m$^3$) en présence de deux potentiels hydriques épidermaux ($\Psi$e ; MPa) de plus en plus négatifs, de- 1, 8 à- 2, 0 à- 2, 2 MPa, attribuables à des apports d'engrais K foliaires et/ou au sol équivalant à ~10% (- 1, 8 à- 2, 0 MPa) et ~20% (- 1, 8 à- 2, 2 MPa) du $K_2O$ mobilisé par la canopée. A noter que ces courbes modèles ne sont pas parallèles et dénotent une plus grande sensibilité de GI et An à [ABA] lorsque $\Psi$e augmente (négativement) .

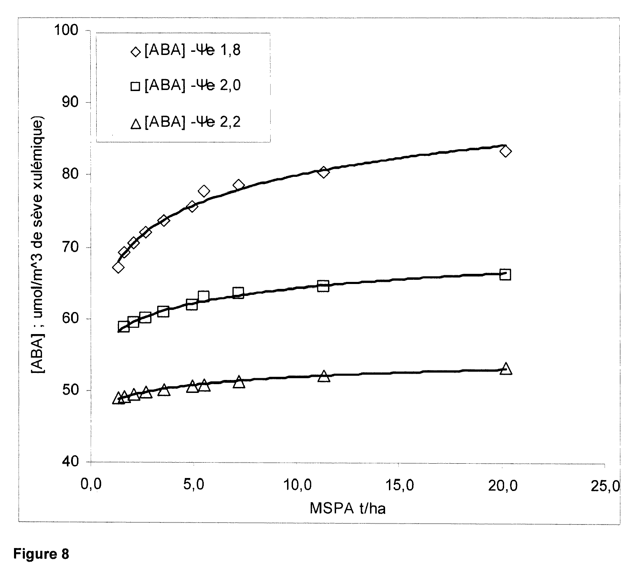

**Figure 8 :** Augmentation logarithmique de la teneur en acide abscissique ([ABA] ; umol/m$^3$) de la sève xylémique et/ou mésophyllienne en fonction de la matière sèche des partie aériennes (MSPA ; t/ha lorsque NNI = 1, 00) d'un blé tendre d'hiver (BTH) pour des potentiels hydriques épidermiques ($\Psi$e ; MPa) de- 1, 8, -2, 0 et- 2, 2 MPa suite à l'apport d'engrais K. L'augmentation négative de $\Psi_e$ de 10% de- 1, 8 à- 2, 0 MPa est effectuée par une apport d'environs 4 à 5 kg- $K_2O$ / ha au feuillage (canopée), par exemple ici d'une blé tendre d'hiver entre les stades BBCH 39 et 55 ; la seconde augmentation à- 2, 2 MPa, soit plus de 20%, implique elle une surdose conjointe d'engrais PK au sol.

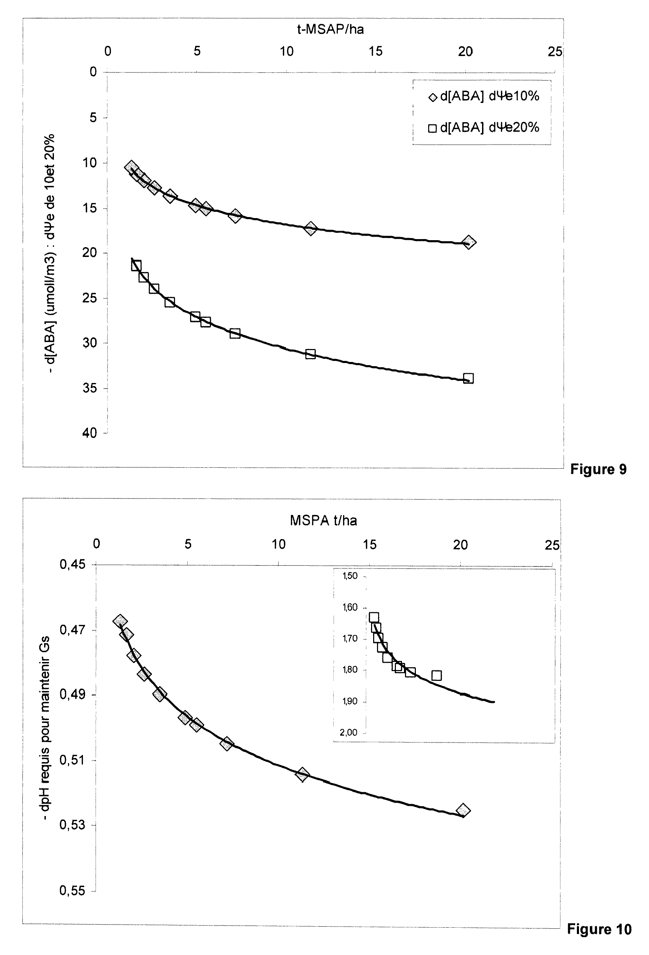

**Figure 9 :** Augmentations logarithmiques (échelle inversée) des différences *effectives* des teneurs en acide abscissique de la sève xylémique et mésophyllienne (d [ABA] ; umol/m$^3$) en fonction de l'accumulation de la matière sèche des partie aériennes (MSPA ; t/ha lorsque NNI = 1, 00) d'un blé tendre d'hiver (BTH) . Ces d [ABA] vont permettre de maintenir GI et An comme si $\Psi$e =- 1, 8 MPa malgré l'augmentation négative de $\Psi$e à- 2, 0, voire à- 2, 2.

**Figure 10 :** Illustration pour le blé tendre d'hiver (BTH) . Augmentation logarithmique (échelle inversée) selon la production de MSPA (t/ha lorsque NNI = 1, 00) des différences effectives des baisses de pH (dpH) des cellules de gardes et/ou para- stomatlques nécessaires afin de maintenir GI et/ou An comme si $\Psi$e =- 1, 8 malgré l'augmentation négative de $\Psi$e à- 2, 0, voire à- 2, 2. Cette première augmentation négative de $\Psi$e de 10% de- 1, 8 à- 2, 0 MPa est effectuée par une apport d'environs 4 à 5 kg- $K_2O$ / ha au feuillage (canopée) entre les stades BBCH 29 et 55 ; la seconde augmentation à- 2, 2 MPa, soit plus de 20%, implique elle une surdose conjointe d'engrais PK au sol.

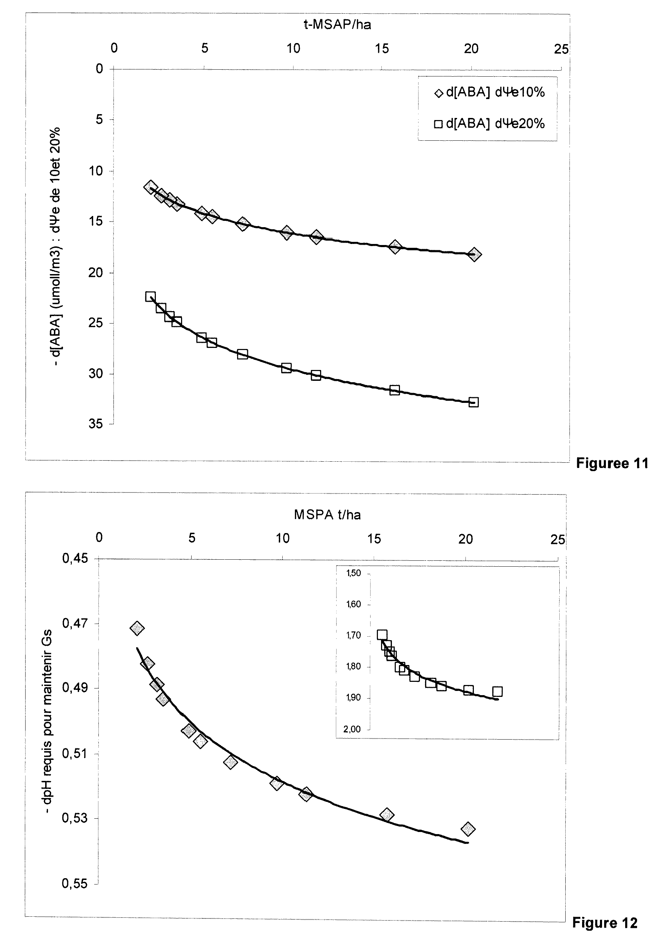

**Figure 11 :** Augmentations logarithmiques (échelle inversée) des différences *effectives* des teneurs en acide abscissique de la sève xylémique et mésophyllienne (d [ABA] ; umol/m$^3$) en fonction de l'accumulation de la matière sèche des partie aériennes (MSPA ; t/ha lorsque NNI = 1, 00) ici et par exemple celle d'un orge d'hiver (ORH) . Ces d [ABA] vont permettre de maintenir Gl et An comme si $\Psi$e =- 1, 8 MPa malgré l'augmentation négative de $\Psi$e à- 2, 0, voire à- 2, 2. Ces d [ABA] sont par rapport au niveau d' [ABA] lorsque $\Psi$e =- 1, 8, soit d'environ 90 umol- ABA/m$^3$ de sève xylémique ; pour la réalisation des $\Psi$e de- 1, 8, -2, 0 et 2, 2 voir les figures précédentes.

**Figure 12 :** Illustration pour l'orge d'hiver (ORH) . Augmentation logarithmique (échelle inversée) des différences effectives des baisses de pH (dpH) des cellules de gardes et/ou para- stomatlques nécessaires afin de maintenir Gl et/ou An comme si $\Psi$e =- 1, 8 malgré l'augmentation négative de $\Psi$e à- 2, 0, voire à- 2, 2. Cette première augmentation négative de $\Psi_e$ de 10% de- 1, 8 à- 2, 0 MPa est effectuée par une apport d'environ 4 à 5 kg- K20 par hectare au feuillage (canopée) entre les stades BBCH 39 et 55 ; la seconde augmentation à- 2, 2 MPa, soit plus de 20%, implique elle une surdose conjointe d'engrais PK au sol.

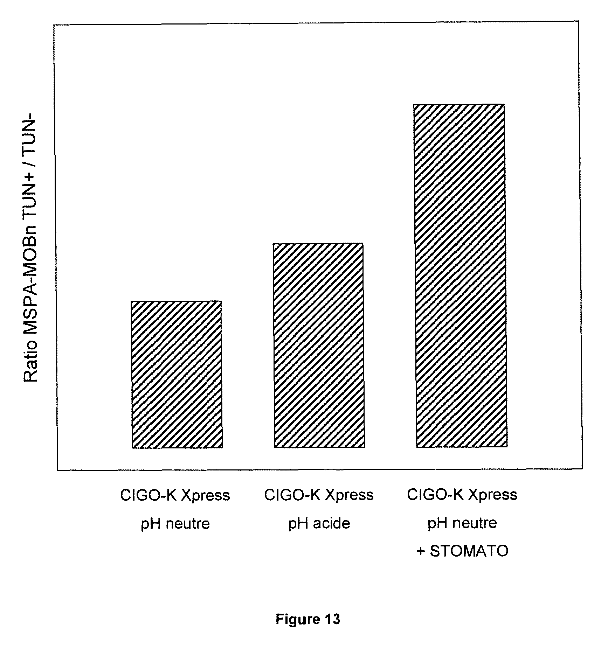

**Figure 13 :** Ratios de la production de matière sèche des parties aériennes (MSPA ; g/pot ou plant) et/ou de leurs mobilisation élémentaire- N ou P (MOBN ou MOBP ; mg/pot) de plantules de *Lolium multiflorum* (Lam) 56 jours post semis à deux taux de fertilisation azote plus (TUN+) ou moins (TUN- ) élevés (mg- Nf/kg- sol) selon le type de modalités CIGO- K Xpress™, soit (i) la formulation commercial à pH neutre (i.e. 6, 7 à 7, 0), (ii) la formulation commercial à pH 6, 3, et (iii) *idem à* (i) mais avec les bactéries STOMATO au sens de la présente invention.

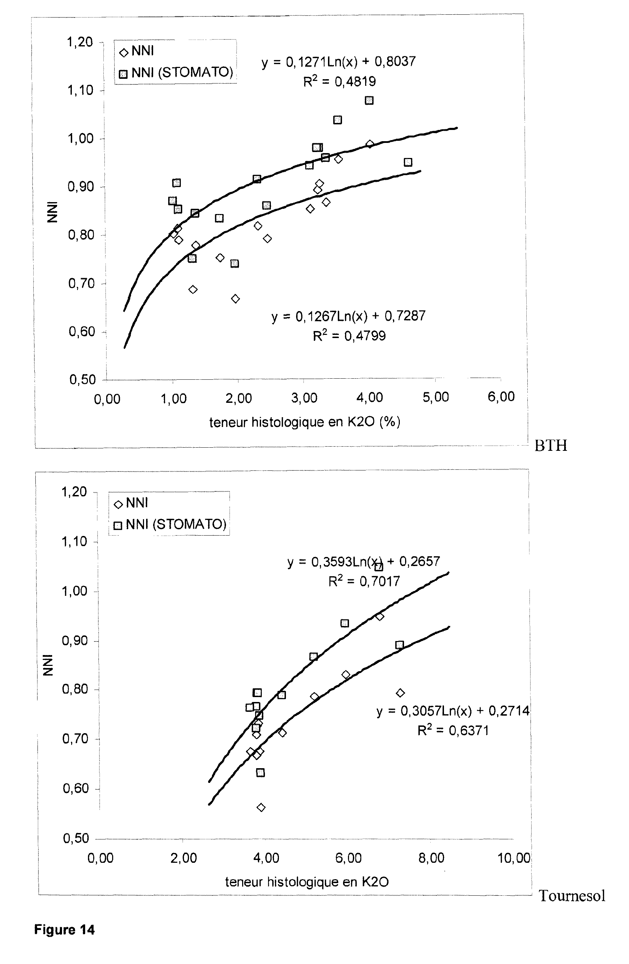

**Figure 14 :** Effets escomptés des bactéries STOMATO sur blé tendre d'hiver (BTH) et tournesol. Pour une même teneur histologique en K$_2$O, l'indice de nutrition azoté (NNI) est plus important avec STOMATO. Concrètement, cela implique qu'il il a plus de matières sèche de produite pour une même teneur histologique en azote ; la synergie NK est donc ici clairement évidente.

## MODE DE REALISATION DE L'INVENTION

La réalisation de l'invention est plausible. Pour le démontrer j'ai du répondre à quatre questions.

1. Les bactéries stomato - philes/taxiques non pathogènes existent-elles ?, et si oui comment les obtenir pour en fabriquer des inocula ?
2. L'apport de 4 à 6 kg-K$_2$O au feuillage étant recherché pour son effet favorable sur l'enzymologie de la synthèse des protéines végétales, quel sera sont impacte $\psi_e$?
3. Une réduction des teneurs en ABA de 10 à 40% sera-t-elle suffisante pour contrecarrer cette augmentation transitoire de $\psi_e$ apte à nuire à la synergie NK ?
4. Quand est-il le plus recommandable d'appliquer l'invention ?

*1. Protocoles d'obtention, de production et d'application des bactéries*

La répartition des bactéries dans ou sur la *phyllosphère* n'est pas uniforme, ou même aléatoire (Lindow et Brandl 2003), mais fonction des sources nutritives (Leveau et Lindow 2001, Miller *et al.* 2001), et/ou des stomates (Delmotte *et al.* 2009, Melotto *et al.* 2006, Hirano et Upper 2000). Cette localisation des bactéries épiphytes sur ou aux alentours des cellules de gardes est donc réelle et facilement reproductible ; les travaux de Chitrakar 2010 et Melotto *et al.* 2008 le démontrent. De plus, la fermeture stomatique que provoque certaines bactéries non pathogène n'est que transitoire, un reflex en sorte, qui ne nuira pas à terme à la conductivité stomatique.

Il y a au moins trois manières d'obtenir des bactéries stomato-philes / taxiques. Les bactéries épiphytes peuvent être isolées de feuillages soit par excision, soit par *fractionnement histologique ;* il est aussi possible d'obtenir directement des biovars mutants de souches bactériennes autrement pathogènes, mais toujours notoirement stomato-philes/taxiques. Dans tous ces cas, il est avantageux d'appliquer une certaines version des postulats de Koch, à savoir ;

1. Les bactéries candidates sont présente dans / sur le feuillage et attroupées alentours des stomates ;
2. Les bactéries candidates sont isolées dudit feuillage et cultivé *in vitro* ;
3. Les bactéries candidates sont de nouveau attroupées alentours des stomates lorsque ré-introduites ;
4. Les bactéries candidates peuvent être à nouveau isolées du nouvel feuillage hôte comportant de tels attroupements puis identifiées comme étant identique aux bactéries candidates en (1).

A) EXCISION DE PELURES EPIDERMIQUES ET OBSERVATIONS ...

L'excision implique ici une manipulation directe sous microscope des cellules de gardes et des bactéries qui leurs sont associées ; voir par exemple le mode opératoire propose par Chitrakar et Melotto 2010 ou encore celui de Trejo *et al.* 1993. Cette association ne doit pas être fortuite mais bel et bien *stomatotaxique* et/ou *stomatophile.* Pour le confirmer, il faut laver les feuilles de manière à exercer une traction susceptible de détacher les bactéries les moins solidement fixées sur ou aux alentours des stomates ; par exemple voir le protocole décrit dans Gudesblat *et al.* 2009. Elles devront par la suite être confirmées comme étant non - pathogènes, acidifiantes et stomatotaxiques et/ou stomatophiles, ayant donc en ce sens avantageusement un effet positif - bien que transitoire, sur l'ouverture des stomates.

L'observation, l'inoculation et l'obtention de bactéries épiphytes capables de migrer vers, voire à travers, des stomates (Gudesblat *et al.* 2009) fait donc partie de l'état de la technique. Ces auteurs ont noté que certaines de ces bactéries sont capables de rétablir l'ouverture, ou moins de contrer la fermeture, des stomates provoquée par l'ABA. Cela dit, certaines de ces bactéries sont pathogènes (Xcc ; op. *cit.*) et n'agissent vraisemblablement pas via une acidification localisée des stomates, mais plutôt via un mécanisme moléculaire impliquant des MAPKinases, voire la *coronatine* (cor ; Melotto *et al.* 2006) . Comme de raison, d'éventuels biovars non pathogènes de telles souches bactériennes sont aussi de bon candidats (*infra, c)* ) .

Plus précisément. Des épidermes, abaxiaux et/ou adaxiaux selon l'espèce de la culture, avantageusement en proximité des veines centrales de la feuille, sont prélevées selon et par exemple Fischer 1968. Des pelures (*peels*, ou *strips*) épidermiques d'environs 0,5 cm de cotés sont délimités par incisions et relevés à l'aide de fine pinces (à épiler) insérées entre l'épiderme en surface et le mésophyll. Ces pelures sont immédiatement mise en flottaison sur un solution appropriée, par exemple constituée des 10 mM de tampon Mes (Sigma Chemicals) et 50 mM KCl, ajustée ≈ pH 6,2 ; voir par exemple les protocoles en ce sens proposées par Trejo *et al.* 1993 et Melotto *et al.* 2006. L'observation de la migration et/ou de l'attachement des bactéries vers les stomates peut être fait selon et par exemple Gudesblat *et al.* 2009. Pour ce faire, il est avantageux d'avoir préalablement transformé un contingent des bactéries candidates appliqué au feuillage avec un plasmide, *eg.* pRU1319, exprimant la protéine fluorescente verte (GFP ; op cit.).

Ces pelures épidermiques peuvent ainsi être incubées quelques heures, eg. 3 heures, lavées d'un jet d'eau pour éliminer les bactéries résiduelles opportunes, et observées directement par microscopie, par balayage laser confocal par exemple (*cf.* Gudesblat *et al.* 2009). Lors de cette observation, les pelures contenant des bactéries *a priori* stomato-philes/taxiques, *i.e.* attroupées alentours des stomates (*cf.* Melotto *et al.* 2006, figure 1 c), sont retenues, voire re-lavées (*supra*) et re-observées ; les susdits postulats de Koch pouvant maintenant être vérifiés, et ainsi de suite ré - itérativement.

**B) EXCISION DE PELURES EPIDERMIQUES ET FRACTIONNEMENT HISTOLOGIQUE ...**

Le fractionnement histologique comporte lui l'excision d'une partie e la feuille, inoculée ou non avec des souches candidates, le broyage en milieux aqueux adéquat (solution tampon) permettant de maintenir la turgescence des cellules de gardes et éviter ainsi leurs lyses. Cette *bouillie histologique* peut être par la suite centrifugée, les cellules de gardes n'ayant pas la même densité que les cellules parenchymes avoisinantes s'accumuleront au sein bande distincte pouvant ainsi être récupérée. L'inoculation d'un milieu de culture solide sélectif avec un aliquote de cette fraction stomatique révélera à terme des bactéries *a priori stomatophiles* et/ou *stomatotaxiques.* Pour confirmer le caractère stomato- phile et/ou- taxique des bactéries candidates il s'agit simplement d'inoculer par pulvérisation l'ensemble de la surface de la feuille (*phylloplan*) , et d'observer par après l'attroupement de celles- ci aux alentours des stomate (*cf.* Melloto *et al.* 2008, Chitrakar *et al.* 2010, Melloto *et al.* 2006, Zhang *et al.* 2008) .

Or, si l'observation des pelures épidermiques est jugée trop fastidieuse, il est aussi possible d'isoler, *i.e.* de séparer, les cellules de gardes stomatiques en acidifiant la susdite solution de flottaison sur laquelle sont placées les pelures épidermiques (*cf.* Squire et Mansfield 1972) . Cette acidification aura pour effet de détruire le fonctionnement des cellules para- stomatiques de l'épiderme et/ou du mésophylle, seules les cellules de gardes stomatiques pouvant par la suite exercer un action sur les bactéries. Une suspension par broyage aqueux de telles pelures, préalablement lavées de leurs bactéries les moins adhérentes, est par la suite diluée en séries (*serial dilution*) et/ou prélevée à l'aide d'un fil à ensemencer par stries une gélose propice au développement de colonies bactériens, en boîtes de Petri par exemple, avantageusement Gram⁻ de la famille *Pseudomonadaceae;* de tels milieux de culture, sélectifs ou non, sont parfaitement connus de l'homme du métier (eg. http: //fr.wikipedia.org/ wiki/ Pseudomonas_ syringae) .

**C) PRODUCTION DE BIOVARS NON PATHOGENES ...**

L'approche la plus directe est d'obtenir simplement un biovar d'une souche bactérienne, avantageusement de *Pseudomonas syringae,* non pathogèneé mais toujours néanmoins stomatophile / -taxique. Par exemple, Mohr *et al.* 2008 décrivent le biovar P. *syringae* 508 ne possédant pas un système d'excrétion de toxines de type III (T3SS) et/ou le gène responsable de la toxine coronatine (COR) autrement présent dans *P. syringae* DC3000 (Melotto *et al.* 2006 ;

Underwood et *al.* 2007). Un tel double mutant de *P. syringae* va donc être doublement phytosanitaire puisqu'il n'y a pas de virulence attribuable à T3SS et d'ouverture forcée des stomates attribuable à la coronatine ; il retiendra cependant son caractère stomato-philique / -taxique. C'est maintenant plus simplement l'acidification localisée à hauteur d'au plus 0,50 unité pH qui va favoriser l'ouverture, ou du moins retarder la fermeture, des stomates sans aucun lien au usuels mécanismes pathogènes (T3SS, COR, etc.) autrement évoqués par P. *syringae.*

Ces biovars non - pathogènes de *P. syringae* peuvent donc êtes eux aussi observés directement sur pelures épidermiques ; elles sont ainsi avantageusement transformée par incorporation plasmidique de la protéine GFP (Melotto et *al.* 2006, Gudesblat *et al.* 2009), voire a priori coronatine négative (cor⁻; Melotto *et al.* 2006) et/ou T3SS négative (T3SS⁻ ; Mohr *et al.* 2008). Les bactéries candidates toujours stomato -philes/taxiques observées sur/dans ces pelures (*supra*) sont elles aussi nécessairement applicables pour la réalisation de la présente invention.

Enfin, à noter que de telles bactéries stomato- philes/ bactériennes peuvent aussi avantageusement appartenir à l'ordre *Actinomycetales,* voire à la familles des *Streptomycetaceae,* certaines espèces membre de cette famille étant reconnues pour leurs qualités phytosanitaire et/ou fongistatiques lorsque attroupées en proximités des stomates.

## D) PRODUCTION DES INOCULA ET APPLICATION AU FEUILLAGE ...

Les bactéries candidates retenues sont produites et formulées, par fermentations soit à l'état solide, soit à l'état liquide à hauteur d'au minimum $10^7$ à $10^8$ cellules bactériennes viables par mL pour une dose - hectare au feuillage de la canopée comprenant entre $10^{11}$ et $10^{13}$ cellules bactériennes viables. Ces isolats candidats peuvent être ré - inoculés et ainsi ré - isolés lors de la vérification des postulats de Koch.

L'application des bactéries au feuillage de la canopée ce fera avantageusement conjointement à un apport d'engrais foliaire (liquide) à base de potassium et d'azote, par exemple CIGO-K Xpress™ (Agronutrition SAS, France). A noter qu'il n'est plus nécessaire d'acidifier la bouillie inoculante. De plus, en principe CIGO-K Xpress™, par exemple, n'a pas d'effet acidifiant sur la bouillie. Si les bactéries en question sont expressément osmotolérantes, il est avantageux de veiller à pré - ajuster l'osmolarité de la bouille inoculante. Afin d'éviter un effet bactéricide, mieux vaux à ce stade introduire la préparation inoculante directement que dans la bouillie ainsi diluées. Il est avantageux d'utiliser un volume d'eau / ha suffisant pour obtenir une répartition homogène sur l'ensemble du feuillage sans ruissellement, et de préférence aux températures comprises entres 6 et 25 degrés C.

*2. Impact physiologique d'apporter des engrais K au feuillage et/ou au sol*

Dewar 2002 rapporte une équation reliant gs, et donc accessoirement Gl, à [ABA] et Ψe qui permet d'apprécier l'impacte déprimant d'une augmentation négative de Ψe sur la conductivité stomatique, gs, et cela pour diverses teneurs en azote du feuillage, Na (g- N/m2) ;

$$g = \frac{a_1(A_\mathrm{n} + R_\mathrm{d})}{c_\mathrm{i}\left(1 + \dfrac{D_\mathrm{s}}{D_0}\right)} \exp\{-[\mathrm{ABA}]\beta \exp(\delta\psi_\mathrm{e})\}$$

    o g : (lire : gs) conductivité stomatique) - mol $CO_2$ / m2 / s  
    o $a_1$ : paramètre combinatoire - sans dimension  
    o $A_\mathrm{n}$ taux d'assimilation (échange) foliaire du $CO_2$ - mol $CO_2$ / m2 / s  
    o $R_\mathrm{d}$ : taux nocturne de respiration foliaire - mol $CO_2$ / m2 / s  
    o $c_\mathrm{i}$ : pression partielle intracelluaire des feuilles - mol $CO_2$ / mol aire  
    o $D_\mathrm{s}$ : déficit de pression partielle de vapeur à la surface foliaire - sans dimension  
    o $D_\mathrm{o}$ : paramètre combinatoire - sans dimension  
    o ABA : concentration xylémique d'acide abcissique - mol ABA / m3  
    o β : paramètre de calibration pour ABA - m3 / mol ABA  
    o $\Psi_\mathrm{e}$ : potentiel hydrique des cellules épidermiques - MPa  
    o δ : paramètre de calibration pour ABA - 1/ MPa  

An est lui fonction de Na (g-N / m2 de feuillage ; Müller *et al.* 2005, 2008) ;

$$A_\mathrm{n} \ (\mathrm{umol} \ / \ \mathrm{m}^2 \ / \ \mathrm{sec}) = (12{,}53 \ \mathbf{x} \ N_\mathrm{a}) - (4{,}44 \ \mathbf{x} \ N_\mathrm{a})$$

Ces valeurs d'An sont ainsi estimées à partir de Na et inscrites dans le modèle selon Dewar 2002, ce qui me permets de construire la Figure 4. A ce stade, les valeurs paramétriques des variables pour cette modélisation semi - empirique Dewar / Müller sont proposées au Tableau 1. Plus la teneur en N du feuillage (Na ; g-N/m2) est importante, plus la conductivité stomatique, gs, bien que toujours grandissante, sera affectée par Ψe fonction du niveau d'apport d'engrais foliaires NK et/ou d'un certain surdosage d'engrais PK au sol.

**Tableau 1 :** Paramètres pour l'équation de Dewar 2002 décrivant gs en fonction de l'augmentation négative de Ψe suite à l'apport d'un engrais (N)K pour diverses teneurs en N (Na) du feuillage. Voir les courbes de ces progressions à la Figure 4 pour des Ψe de -1,0 à -2,5.

| Paramètre | unité | valeur |
|---|---|---|
| a1 | na | 30 |
| An : m | Mol-$CO_2$/m$^2$/s | de 6 à 28 |
| Rd | mol-$CO_2$/m$^2$/s | 0 |
| ci | umol-$CO_2$/mol-aire | 288 |
| Ds | na | 0,0330 |
| Do | na | 0,01000 |
| ABA | umol-ABA/m$^3$ | de 75 à 150 |
| Ψe | Mpa | de -1,0 à -2,5 |
| β | m$^3$/mol-$H_2O$ | 1,48E-04 |
| δ | 1/Mpa | -2 |

Dans les faits, cette modulation de Ψe (dΨe) sera de l'ordre de 5 à 20%, avantageusement d'environs 10%. En effet, des quelques 200 à 250 kg-$K_2O$/ha mobilisés par la MSPA d'une céréales à paille d'hiver telle que le blé tendre d'hiver entre les stades BBCH 35 et 61, plus de la moitié sera dans les tiges, et moins de l'autre moitié dans le feuillage de la canopée la plus accessible par une pulvérisation liquide des susdits engrais foliaire NK. Grossièrement, le feuillage de la canopée à de tels stades de croissance représente donc environ 25% de la MSPA. Puisqu'il est possible d'apporter de 4 à 6 kg par hectare de $K_2O$ au feuillage via des engrais NK de type Cigo K-Xpress™ (Agronutrition SAS), de tels apports de K représentent bel et bien environ 10% du $K_2O$ mobilisé ; un dΨe attribuable à une telle augmentation de K est donc parfaitement plausible.

*3. Les réduction d[ABA] effectives associée à de tels d'Ψe sont elles plausibles ?*

Cette baisse (augmentation négative) de Ψe, même de l'ordre de 10 à 20%, va donc nuire à la synergie KN puisque dΨe réduira gs d'autant plus rapidement que Na est important. Pour rétablir gs, et donc An, il faut réduire [ABA]. Mais de combien ? Est-il effectivement plausible de réaliser cette réduction d'[ABA] via une acidification localisée de l'ordre de 0,33 à 0,67 unité pH commensurable avec le pouvoir acidifiant d'une bactérie ? Pour y répondre, j'ai fait appel à un modèle informatisée du fonctionnement stomatique au niveau foliaire dit LEAFC3N dont le pilotage est rapporté dans Müller *et al.* 2005 et 2008.
*A priori* LEAFC3N peut facilement modéliser l'augmentation de gs selon celle de Na et An. Cela dit, cette simple modélisation fixe en sorte Ψe ce qui est irréelle ici puisque l'apport d'engrais K foliaire va de facto augmenter Ψe d'environs 10%, soit vraisemblablement ici et à titre d'exemple de -1,8 à -2,0, voire -2,2 MPa. Il faut donc moduler la valeur de Ψe dans le module de pilotage de LEAFC3N (start_LC3N_sample_scalar; Müller et Eschendöder 2012), et incrémenter Na de 0,80 à 3,50, dans le cas du blé tendre d'hiver (BTH ; Tableau 2) et de 0,75 à 2,50 g-N/m2 dans le cas de l'orge d'hiver (ORH).
Selon la documentation de LEAFC3N (Müller et Eschendöder 2012), les variables du modèle sont ;

- An : taux d'échange (foliaire) net du $CO_2$ (umol/m$^2$/sec)
- Ci : concentration du $CO_2$ sous stomatique et/ou dans les cavités intercellulaire (umol/mol)
- gs : conductivité stomatique au $CO_2$ (mol/m$^2$/sec)
- Gl : conductivité stomatique au $CO_2$ (mol/m$^2$/sec), ... au niveau foliaire
- m : coefficient du model BWB décrivant la reaction de gs à An (sans unitées)
- Na : masse d'azote (N) par unité de surface foliaire (g-N/m$^2$)
- Rd : taux de respiration diurne en présence d'un flux photonique donnée (umol/m$^2$/sec)
- β : coefficient décrivant la souplesse de transition entre échange et fixation du $CO_2$ (sans unitées)
- Ψe : potentiel hydrique de la feuille (épiderme, ou phylloplan ; Pa).

A noter qu'a ce stade, LEAFC3N, du moins l'actuelle version scalaire, ne re-calcul automatiquement la teneur en chlorophylle du feuillage en fonction de Na. Il faut donc le faire préalablement selon la relation suivante, pour le blé tendre d'hiver (Evans 1983), et incrémenter ces valeurs en parallèle à celles de Na ;

$$Chl = 33,6Nc2 + 361Nc + 0,88 \ (umol \ Chl_a \ / \ m2)$$

A note aussi r qu'à l'aide de LEAFC3N il est maintenant possible de modéliser directement An. Il est donc possible de *rétro-calculer* [ABA] à l'aide de la formule de Dewar 2002 pour chacune des valeurs de Na et Chl ;

$$[ABA] \ = \ \frac{(log(gs) \ / \ \beta exp(\delta \Psi e))}{a1 \ [log((a1(An + Rd) \ / \ ci(1+Ds/Do))]}$$

Selon cette modélisation, hybride en sorte, les progressions de Gl et An selon Na sont ralenties par une augmentation négative de Ψe de -1,8 à -2,2 (Figure 5). Paradoxalement, [ABA] ne progresse pas selon Na (Figure 6) ; c'est plutôt la sensibilité de gs et An à [ABA] qui est plus grande si Ψe passe de -1,8 à -2,2 (Figure 7).

Pour maintenir Gl et/ou An lors d'une augmentation de Ψe de l'ordre de 10 à 20% attribuable à un engrais foliaire NK, il faut réduire [ABA]. Cette réduction sera plus limitée si Gl, ou gs et/ou An sont important, c'est-à-dire si Na l'est aussi (Figure 5). En d'autre mots ; un dΨe de 10% rend Gl et An plus sensibles à [ABA], d'où l'importance de réduire [ABA] si Ψe augmente suite à l'apport d'un engrais K. Cette sensibilité de gs et An à [ABA] dépends aussi de la MSPA. [ABA] progresse logarithmiquement selon l'augmentation de la MSPA (Figure 8) ; l'écart entre ces [ABA] à différents Ψe va donc croître à mesure que s'accumule la MSPA. Inversement, le d[ABA] à réaliser pour maintenir immédiatement gs et/ou An malgré une augmentation négative de Ψe va lui aussi croître avec MSPA (Figure 9) ; il deviendra en principe plus difficile de réaliser l'effet recherché à mesure que progresse MSPA.

**Tableau 2 :** Paramétrage des variables de la modélisation hybride LEAFC3N combinant un rétro-calcul des teneurs en ABA des sèves xylémiques à l'aide de l'équation décrit par Dewar 2002 à trois deux différant potentiel hydriques épidermaux Ψe tels qu'affectés par l'application d'engrais K foliaire et, pour les Ψe les plus négatifs (*i.e.* -2,2 MPa), d'une surdose d'engrais PK au sol. Il s'agit ici d'une modélisation pour le blé tendre d'hiver (BTH) ; la modélisation pour l'orge d'hiver (ORH) donne essentiellement les mêmes résultats.

| Na | Chl | Ψe | Gs | Gl | An | Rd | Ci | [ABA] |
|---|---|---|---|---|---|---|---|---|
| g/m2 | umol/m2 | Pa | mol-CO2/m2/s | mol-CO2/m2/s | umol-CO2/m2/s | umol-CO2/m2/s | umol-CO2/mol | umoll/m3 |
| 0,80 | 268 | -1,8 | 0,165 | 0,158 | 7,15 | 0,176 | 285 | 90,8 |
| 1,00 | 328 | -1,8 | 0,187 | 0,179 | 8,92 | 0,226 | 278 | 87,1 |
| 1,25 | 399 | -1,8 | 0,212 | 0,201 | 11,03 | 0,289 | 271 | 83,4 |
| 1,50 | 466 | -1,8 | 0,233 | 0,220 | 13,05 | 0,352 | 264 | 80,3 |
| 1,67 | 510 | -1,8 | 0,246 | 0,232 | 14,38 | 0,394 | 259 | 78,6 |
| 1,75 | 529 | -1,8 | 0,252 | 0,237 | 14,99 | 0,414 | 257 | 77,8 |
| 2,00 | 588 | -1,8 | 0,269 | 0,252 | 16,86 | 0,477 | 252 | 75,6 |
| 2,25 | 643 | -1,8 | 0,285 | 0,265 | 18,64 | 0,540 | 247 | 73,7 |
| 2,50 | 693 | -1,8 | 0,299 | 0,277 | 20,36 | 0,603 | 242 | 72,0 |
| 2,75 | 739 | -1,8 | 0,312 | 0,288 | 22,01 | 0,665 | 237 | 70,6 |
| 3,00 | 781 | -1,8 | 0,324 | 0,298 | 23,59 | 0,728 | 233 | 69,3 |
| 3,50 | 852 | -1,8 | 0,344 | 0,316 | 26,56 | 0,854 | 225 | 67,1 |
| 0,80 | 268 | -2,0 | 0,133 | 0,128 | 6,50 | 0,176 | 277 | 70,4 |
| 1,00 | 328 | -2,0 | 0,150 | 0,144 | 8,06 | 0,226 | 269 | 68,3 |
| 1,25 | 399 | -2,0 | 0,168 | 0,161 | 9,94 | 0,289 | 260 | 66,2 |
| 1,50 | 466 | -2,0 | 0,184 | 0,176 | 11,72 | 0,352 | 252 | 64,5 |
| 1,67 | 510 | -2,0 | 0,194 | 0,185 | 12,89 | 0,394 | 247 | 63,5 |
| 1,75 | 529 | -2,0 | 0,199 | 0,189 | 13,43 | 0,414 | 245 | 63,1 |
| 2,00 | 588 | -2,0 | 0,211 | 0,200 | 15,06 | 0,477 | 239 | 62,0 |

(suite)

| Na | Chl | Ψe | Gs | GI | An | Rd | Ci | [ABA] |
|---|---|---|---|---|---|---|---|---|
| g/m2 | umol/m2 | Pa | mol-CO2/m2/s | mol-CO2/m2/s | umol-CO2/m2/s | umol-CO2/m2/s | umol-CO2/mol | umoll/m3 |
| 2,25 | 643 | -2,0 | 0,223 | 0,211 | 16,61 | 0,540 | 233 | 61,0 |
| 2,50 | 693 | -2,0 | 0,233 | 0,220 | 18,10 | 0,603 | 227 | 60,1 |
| 2,75 | 739 | -2,0 | 0,242 | 0,228 | 19,52 | 0,665 | 222 | 59,4 |
| 3,00 | 781 | -2,0 | 0,251 | 0,235 | 20,88 | 0,728 | 217 | 58,8 |
| 3,50 | 852 | -2,0 | 0,266 | 0,248 | 23,44 | 0,854 | 209 | 57,8 |
| 0,80 | 268 | -2,2 | 0,105 | 0,102 | 5,81 | 0,176 | 267 | 54,3 |
| 1,00 | 328 | -2,2 | 0,118 | 0,114 | 7,17 | 0,226 | 258 | 53,2 |
| 1,25 | 399 | -2,2 | 0,131 | 0,127 | 8,79 | 0,289 | 248 | 52,2 |
| 1,50 | 466 | -2,2 | 0,143 | 0,138 | 10,32 | 0,352 | 239 | 51,3 |
| 1,67 | 510 | -2,2 | 0,150 | 0,144 | 11,32 | 0,394 | 234 | 50,9 |
| 1,75 | 529 | -2,2 | 0,153 | 0,147 | 11,78 | 0,414 | 231 | 50,7 |
| 2,00 | 588 | -2,2 | 0,162 | 0,155 | 13,16 | 0,477 | 224 | 50,1 |
| 2,25 | 643 | -2,2 | 0,170 | 0,163 | 14,46 | 0,540 | 217 | 49,7 |
| 2,50 | 693 | -2,2 | 0,177 | 0,169 | 15,70 | 0,603 | 211 | 49,4 |
| 2,75 | 739 | -2,2 | 0,183 | 0,175 | 16,87 | 0,665 | 205 | 49,2 |
| 3,00 | 781 | -2,2 | 0,189 | 0,180 | 17,97 | 0,728 | 200 | 49,0 |
| 3,50 | 852 | -2,2 | 0,198 | 0,189 | 20,02 | 0,854 | 190 | 48,8 |

*Nb.* : Valeurs paramétrique pour l'équation selon Dewar 2002 permettant de rétro - calculer les teneurs en ABA ([ABA]).

| β | δ | a1 | Ds | Do |
|---|---|---|---|---|
| m3/mol -ABA | M pa | N/A | Mpa | Mpa |
| 1,00E-04 | - 1,80 | 3,30E-02 | 1,80E-05 | 1,75E-05 |

Enfin, il est aussi possible de reformuler le problème en terme de d [ABA] réalisable, ou non, soit ici de l'ordre de 10 à 35 umol- ABA / m3 de sève (Figure 9) . À l'aide de la formule suivante issue des données de Wilkinson et Davies 2008 ;

$$\%[ABA]\ nmol/g\text{-}MSPA : maxima = 16 \times (pH)^2 - 167 \times (pH) + 499\ (R^2 = 0,43)$$

il est ainsi possible de transformer ces d [ABA] en dpH capables de les effectuer (Figure 10) . Or, . pour une dΨe de 10%, soit de Ψe- 1, 8 à- 2, 0, ces dpH sont parfaitement réalisable par voie d'acidification localisée des cellules de gardes et para- stomatiques de blé tendre d'hiver, soit ici de l'ordre d'à peine une demi unité pH (Figure 10) ; un dΨe de 20%, soit de- 1, 8 à- 2, 2 est lui par contre beaucoup plus difficile à atténuer, car il implique une diminution du pH (para) stomatique par  plus d'une unité et demi (abîme Figure 10) . J'aussi réalisé ces même analyses via LEAFC3N pour l'orge d'hiver (ORH ; Figures 11 et 12) .

C'est donc surtout la sensibilité de GI (ou gs) et An aux concentrations xylémiques et mésophylliennes d'ABA ([ABA]) qui augmente si ψe augmente (négativement) suite à un apport de 4 à 6 kg-K$_2$O / ha au feuillage. Inversement, une réduction de [ABA] par voie d'acidification locale à l'aide de bactéries stomato-philes/taxiques (STOMATO) sera d'autant plus marquée puisque la *réactivité,* par opposition à une simple *sensibilité,* de gs et An à de telles diminutions d'[ABA] est beaucoup plus grande si ψe est de -2,0 plutôt que de -1,8 MPa. Le taux d'accroissement d'ABA par rapport à gs est de plus en plus important à mesure qu'augmente (négativement) ψe.

Or, c'est précisément cette accélération que cherche à atténuer l'invention. Pour ce faire, il faut que [ABA] diminuer, avantageusement bien en dessous d'environ100 umol/m$^3$ de feuillage (*eg.* Borel et Simoneau 2002) . Cette réduction du seuil de tolérance à [ABA] de 30 à 40% si ψe augmente illustre bien le problème technique ; l'apport d'engrais K foliaire capables de rendre immédiatement mis transitoirement plus négatif ψe oblige en sorte de ramener, de maintenir, [ABA] en dessous d'un seuil préalablement, lire : sans l'apport d'engrais K foliaire, plus élevé. Ne pas tenir compte de cet impacte immédiat et transitoire d'engrais foliaire K sur ψe explique en bonne partie pourquoi la synergie NK se dégrade si rapidement en de telles circonstances.

Les bactéries stomato-philes/-taxiques seront aussi plus efficaces si Ψe est plus négatif suite à un apport foliaire d NK. Leur effet provient d'une réduction d'[ABA] en accord avec les besoins de gs et/ou An, mais aussi d'une plus grande *réactivité* de gs et/ou An suite à de telles réductions d'[ABA]. Ici et par exemple, pour maintenir GI (ou gs) et/ou An en présence d'engrais K foliaires, [ABA] doit être ~70 plutôt que ~100 umol par $m^3$ ; cette réduction d'[ABA] est plausible via ces bactéries capables de réduire le pH de cette sève, ou plus particulièrement ici des cytosols des celles de gardes et/ou para - stomatiques, d'à peine une 1/2 de pH.

Synthétiquement, l'effet des bactéries stomato- philes/ taxiques apportées conjointement à un engrais foliaire NK peut être décrit comme suite ;

1. L'apport de K va changer les relations gs, GI et An = f([ABA]) en déplaçant les courbes vers la gauche des graphes à la Figure 7.

2. Cela peut provoquer immédiatement mais transitoirement une chute de gs, GI et/ou An en raison de l'augmentation de ψe (potentiel hydrique des cellules épidermiques) plutôt qu'exclusivement de Ψg (potentiel hydrique des cellules de gardes).

3. Lesdites bactéries vont entre en jeu et réduire [ABA] via une acidification de l'ordre de 0,33 à 0,67 unité de pH, soit suffisamment pour situer [ABA] dans le cadre de la relation gs, GI et/ou An = f([ABA]) lorsque ψe ≈ -1,1.

4. Enfin, en réponse à une telle baisse d'[ABA], les valeurs de gs, GI et/ou An vont remonter à leurs niveaux d'avant l'apport d'engrais K.

Tout cela ce passe dans l'intervalle de temps nécessaire au K apporté aux cellules épidermiques pour migrer activement vers les cellules de gardes et/ou para - stomatiques. STOMATO ne fait que réduire ce intervalle pendant lequel ψe ainsi rendu plus négatif est contreproductif en terme de gs, GI et/ou An, faute de quoi, la synergie NK tant recherchée sera affectée.

*4. Quand faut-il appliquer l'invention ?*

Il est avantageux d'appliquer cet engrais bactérien à base d'azote et de potassium lorsque NNI est plus grand ou égale à 1,00, c'est à dire quand la teneur en azote du feuillage n'est pas *a priori* préjudiciable au plein rendement de la culture. En effet, lorsque NNI = 1,00 Nc = Nh, *i.e.* les teneurs pondérales en N des parties aériennes de la culture (% ; p/p) établie comme critique (Nc) selon la fonction empirique suivant, par exemple ici pour le blé tendre d'hiver (BTH ; Gastal et Lemaire 2002, Jeuffroy *et al.* 2002) ;

$$Nc = a \times (MSPA)^{-k} \text{ ; } a = 5,35 \text{ et } k = 0,442$$

valeur critique en dessous de laquelle la production optimale de biomasse est compromise, est égale à Nh (% ; p/p) la teneur *histologique* effectivement mesurée à ce stade d'accumulation de la MSPA. On peut ainsi recalculer MSPA si NNI = 1,00 de la sorte (pour le blé d'hiver) ;

$$MSPA = 10^{[log(N\text{-}MSPA/a)]/\text{-}k}$$

A noter que pour convertir en Nh les valeurs de Na (g-N / $m^2$ de feuillage) issues des modélisation LEAFC3N, il est possible d'utiliser la fonction empirique suivante, pour le blé tendre d'hiver, voire faute de mieux pour la plupart des céréales, tirée de Müller *et al.* 2005, 2008 ;

$$Na = a_N \times N_h - b_N \text{ } (R^2 = 87\%)$$

$$a_N = 60,79 \text{ g-N} / m^2 \text{ ; } b_N = 0,1381 \text{ g-N} / m^2$$

*(i) en fonction des stades BBCH de développement de la culture :*

Les dpH (Figure 10) et d[ABA] (Figure 9) à atteindre afin de maintenir GI et An à des niveaux comparables à ceux avant l'apport d'engrais foliaire NK varient à travers la campagne en fonction de l'accumulation de la MSPA. De manière générale, il est plus facile d'obtenir le résultat escompté si dψe ne dépasse pas 10% et avant que la MSPA ne soit trop abondante. Par exemple ici avec un blé (Figures 9 et 10) ou d'un orge d'hiver (Figures 11 et 12) avant que le stade des 10 t-MSPA par hectare ne soit franchi.

En effet, si NNI = 1,00, $N_{MSPA}$ = Na, tandis que ;

$$Nc = a \times (MSPA)^{-k} ; a = 5{,}35 \text{ et } k = 0{,}442 \ (cf. \text{ Gastal et Lemaire 2002}).$$

On peut ainsi recalculer MSPA si NNI = 1,00 de la sorte (pour le blé d'hiver) ;

$$MSPA = 10^{[log(N-MSPA/a)]/-k}$$

Les valeurs usuelles de a et k pour différentes cultures C3 et C4, y compris l'orge d'hiver, sont elles rapportées dans Gastal et Lemaire 2002 et/ou Jeuffroy *et al.* 2002. Connaissant donc approximativement la MSPA, il est possible de cerner la date et/ou le stade BBCH atteint. Pour ce faire, j'ai utilisé une simple régression linéaire décrivant la MSPA post 6 avril en régions tempérées, soit ici et par exemple en France au nord de Loire ;

$$MSPA \text{ (kg/ha)} = 187 \times [\text{jours post 6 avril}] + 918$$

Connaissant ainsi la MSPA produite si NNI = 1,00 il est aussi possible d'estimer le nombre de jours, disons ici post 6 avril, pour atteindre cette MSPA, et donc approximativement le stade de croissance BBCH à cette date. Par exemple, pour une céréale à paille en Europe de l'Ouest, la MSPA produite à divers stade BBCH est approximativement la suivante (Tableau 3) ;

**TABLEAU 3 :** Production *approximative* de matières sèches des parties aériennes (MSPA) d'une céréale à paille en Europe de l'ouest à certains stades BBCH, leurs dates juliennes approximatives et les teneurs en Nh à ces stades / dates lorsque NNI = 1,00

| BBCH | MSPA (t / ha) | ~ date | ~ %Nh |
|------|---------------|--------|-------|
| 26-27 | 0,11 | 30-mars | 5,50 |
| 27-28 | 0,27 | 31-mars | 5,11 |
| 28-29 | 0,68 | 2-avr. | 4,24 |
| 29-30 | 1,84 | 8-avr. | 3,37 |
| 30-31 | 5,43 | 28-avr. | 2,50 |
| 31-33 | 7,24 | 7-mai | 2,28 |
| 33-37 | 9,70 | 20-mai | 2,06 |
| 37-39 | 11,89 | 1-juin | 1,91 |
| 39-59 | 13,10 | 8-juin | 1,85 |
| post 61 | > 15 | post mi-juin | 1,63 |

Bien que les Figures 9 à 12 laissent entendre qu'il vaut mieux intervenir plus tôt que tard dans la saison, faute de quoi les d[ABA] et dpH requis pourraient devenir plus difficilement réalisables, il faut nuancer. En effet, à l'automne, après la récolte et en présence de résidus de culture pailleux au sol les teneurs des sols en azote minéral (kg-$N_m$/ha) seront faibles et la synergie NK est inaffectée. Cela suggère la préconisation d'une application foliaire plus tardive, dès la sortie d'hiver et après les premiers apports d'azote fertilisant pouvant augmenter les teneurs en azote histologique (Nh) de façon à nuire à la synergie NK lorsque NNI > 1,00.

Les teneurs des sols en azote minéral à travers l'inter - culture (automne → printemps) peut elle aussi affecter la réalisation de l'invention. Plus précisément, et en termes de taux de fertilisation azotée (TUN ; kg-N par hectare), une teneur plus ou moins ponctuelle des sols de l'ordre de 125 à 150 kg-N par hectare (sur 20 cm de profondeur pour une densité apparente de 1,25) pourrait atténuer la synergie NK et c»ontribuer à mettre en évidence la réalisation de l'invention. Or, à travers la campagne céréalière automne/hiver/printemps, ces seuils ne sont pas nécessairement franchis systématiquement.

Le diagnostique intra- saisonnier de l'état nutritionnel azoté de la culture peut aussi servir à établir quand appliquer l'invention. Par exemple, si un certain seuil critique en azote nitrique a été franchi rendant ainsi l'acidification localisée des apoplastes et parenchymes stomatiques nécessaire et utile. Plus particulièrement et par exemple, les seuils critiques en nitrates des jus de bas de tiges (jbt) d'un blé tendre d'hiver selon la méthode Jubil™ (INRA- Arvalis) sont avantageusement entre 1250 et 2000 mg/L, soit de 20 à 32 moles. Ces concentrations en nitrates seront fonction des TUN et de l'ensemble des fournitures en N du sol, ce dernier pouvant être avantageusement modélisé à partir des valeurs des reliquats d'azote à l'automne, de la teneur en matière organique du sol et de son taux de minéralisation. Nous estimons que des fournitures annuelles en Nm, y compris via les apports d'Nf, par le sol de l'ordre de 250 kg-N/ha pour les céréales, et de 320 kg- N/ha pour le maïs- grain peuvent provoquer une dégradation de la synergie NK et rendre ainsi utile voire nécessaire la réalisation de la présente invention.

## EXEMPLES D'APPLICATION AGRONOMIQUE

La synergie NK elle pourra être assurée et comparée en pratiquant deux TUN - supra et infra-optimaux, la susdite acidification localisée étant en principe relativement plus efficace (par rapport à l'acidification généralisée) à haut TUN, là ou son inhibition de l'activité de l'ABA est à pressentir comme la plus efficace. Donc, dans un premier temps, nous suggérons à ce stade un dispositif avec maïs en parcelles imbriquées, la parcelle principale étant le niveau de TUN (-/+), avec chacune des trois (3) modalités suivantes (Figure 13) ;

➢ CIGO-K Xpress™ tel que formulé commercialement
➢ CIGO-K Xpress™ acidifié, mais non bactérisé
➢ CIGO-K Xpress™ bactérisé, mais non acidifié

Afin de réaliser TUN- et TUN+, il faut simplement reprendre les molarités de $KNO_3$ proposées par Wilkinson *et al.* 2007, soit l'équivalent de 50 (TUN-) et 125 (TUN+) mg-N par L de solution sur support de vermiculite, voire de 67 et 133 mg-N / kg de sol reconstitué, respectivement. Dans un deuxième temps, préparations STOMATO auront un effet bénéfique sur la synergie NK via une amélioration de la conductivité stomatique, gs. Cette amélioration de la synergie NK sera a priori le plus aisément détectable via la mesure de *l'efficacité relative* (er) d'un engrais K foliaire comme CIGO-K Express™ à différents taux de fertilisation N, et donc du coup à différentes teneurs en N du feuillage. Cette efficacité relative est ici le ratio des MSPA des deux modalités avec et sans engrais K foliaire appariés au sein d'un dispositif en carré latin 5 x (5 x 2) comprenant cinq (5) taux de fertilisation N - disons de 0 à 100 mg-Nf / kg-sol par tranche de 20 mg-N, soit approximativement de 0 à 200 kg-Nf par hectare. Ce dispositif sera reproduit côte à côte deux fois avec et sans lesdites préparations STOMATO ; les résultats escompté sont illustrés à la Figure 7.

*In situ* (au champ), l'effet stimulant de l'invention sur la synergie NK peut être apprécié *via* l'augmentation de NNI pour une même teneur histologique en $K_2O$ (%) de plants prélevés individuellement à travers la campagne ; à la Figure 14 je propose quelques simulations pour le blé tendre d'hiver (BTH) et le tournesol. Le déplacement vers le haut de la courbe NNI (STOMATO) implique que pour une même teneur histologique en $K_2O$ la plant aura produit plus de MSPA et requière donc moins d'azote pour assurer son plein rendement de biomasse à ce stade, d'où un NNI = Nh / Nc, Nc étant ici ce seuil critique (g-N / kg) requis et Nh la teneur histologique en N (g-N / kg) effectivement mesurée. Or, la synergie NK implique que le potassium sert surtout de cofacteur enzymatique lors de la protéogénèse qui elle même régulera le taux de mobilisation de l'azote ; plus cette mobilisation de l'azote est importante, plus ladite protéogénèse le sera aussi. En effet, le potassium aura pu lui être simplement prélevé de manière luxuriante sans pour autant contribuer à cette mobilisation de l'azote préalable à ladite protéogénèse ; ce phénomène de consommation luxuriante du potassium est bien connu de l'homme du métier.

Enfin, la production d'ABA et de NO étant liée aux stresses, hydriques notamment, l'invention est pertinente dans au moins deux cas de figures ;

• s'il y a stresses, les bactéries stomato - philes/taxiques vont assurer une [ABA] plus en accord avec le fonctionnement *ad minima* des engrais NK ;
• s'il n'y a pas de stresses, les bactéries stomato - philes/taxiques vont être particulièrement efficaces à augmenter GI et/ou An en raison de la très (plus) forte réactivité de GI et An à des Ψe plus faibles.

Cet aspect de l'invention est conséquent pour la commercialisation des engrais foliaire dont le supposément principal avantage est son utilisation en cas de sécheresses lors desquelles l'absence d'eau au sol empêche la dissolution des granules d'engrais apportées au sol. Les engrais foliaire, eux, ne nécessitant pas un telle dissolution sont généralement considérés comme plus assimilables en de telles circonstances. Or, s'il y a sécheresse, il y aussi stresses hydriques et donc fermeture des stomates qu'un quelconque engrais NK foliaire ne fera qu'exacerber. D'où l'utilité de la présente invention qui permet atténuer légèrement cette fermeture, préventive c'est vrai, des stomates.

## SIGLES ET DEFINITIONS

**Canopée :** Portion feuillue de la matières sèche des parties aérienne (MSPA) d'une culture représentant l'étage sommital de cette biomasse la plus accessible à un pulvérisation liquide d'un engrais foliaire et/ou un quelconque produit phytosanitaire. Grossièrement, le feuillage de la canopée représente envions 25% de la MSPA entre les stades BBCH 35 et 61.

**BFCP :** bactéries favorisant la croissance des plantes (*alias* PGPR : *plant growth promoting rhizobacteria*)

**Ψe :** potentiel hydrique des cellules épidermiques et/ou para - stomatiques - MPa

**ψg :** potentiel hydrique des cellules stomatiques - MPa

**Stomatotaxique :** faculté qu'on certains microorganismes - et plus particulièrement ici bactériens, à se mouvoir à travers le phylloplan vers les cellules de gardes stomatiques (stomates)

**Stomatophilique :** faculté qu'on certains microorganismes - et plus particulièrement ici bactériens, à s'ancrer sur ou en proximité des cellules de gardes stomatiques (stomates)

**Cellules de gardes :** cellules réniformes qui délimitent l'orifice stomatique

**Parenchymes :** cellules des tissus végétaux constitués de cellules (vivantes) pecto-cellulosique perforées de plasmodesmes favorisant une circulation des substances à l'intérieur des cellules (symplastes).

**Bouillie histologique :** mélange de cellules de la phyllopsphère une fois celles-ci détachées les unes des autres, par malaxage (mixage) mécanique par exemple. Il s'agit ici surtout de provoquer une séparation des cellules de gardes des parenchymes (*i.e.* épidermiques et/ou para - stomatiques).

**Apoplaste :** ensemble des interstices et parois cellulaires des cellules mortes du xylème ; l'eau et les solutés empruntent cette voie pour s'infiltrer jusqu'aux organes sans pénétrer dans une cellule

**Symplaste :** ensemble des cytoplasmes cellulaires en réseau *via* de petits tunnels qui perforent les parois entre les cellules (plasmodesmes)

**Fractionnement histologique :** séparation physico-chimique et/ou mécanique des cellules de gardes et para - stomatiques des cellules parenchymes. Ce fractionnement devra ici permettre

**Phyllosphère :** ensemble de la surface de la feuille, y compris les celles parenchymes et stomatiques immédiatement sous la cuticule.

**Phylloplan :** surface de la feuille.

**ABA :** acide abcissique

**NR :** nitrate réductase

**ANN :** agents azoto-nutritonnel

**Synergie NK :** efficacité relative croissant des engrais N qu'en présence d'un optimum d'engrais K

**Seuil (N) infra :** teneur en *Na* à partir de laquelle l'augmentation la synergie NK plafonne.

**Seuil** (N) **supra** : teneurs en *Na* à partir de laquelle la synergie NK décroit

**Nc - Azote critique** (mg-N / g-MSPA, ou en %)

**Nm - Azote minéral** (mg-N / kg-sol) : teneur en Nm du sol

**Nf - Azote fertilisant** (kg-N / ha) : apport en N au sol et/ou au feuillage

**Nh - Azote histologique** (mg-N / g-MSPA, ou en %)

**Na - Azote du feuillage** (mg-N / $m^2$ de feuillage)

**Apports qualités :** quantité d'engrais N (Nf) apporté en fin de cycle de production céréalière afin d'assurer un minimum de teneur en protéines des grains - cette apport est souvent de l'ordre de 40 kg-N par ha.

**SAU :** superficie agricole utile (hectare ; ha)

**TUN :** taux de fertilisation azotée (kg-Nf / ha)

**Dose-X** (d'azote fertilisant, Nf ; kg-Nf/ha) : apport d'Nf préconisé comme optimal. Celle-ci tient compte des reliquats d'azote à l'automne, des fourniture d'Nm par la matière organique du sol, etc. Elle est obtenue via une modélisation et/ou via un pilotage intra-saisonnier des apports fractionnés de Nf. **Jubil™ :** méthode commercialisée de diagnostique de l'état nutritionnel de la plante et de pilotage intra-saisonnier de la fertilisation NPK,

**Jus de base de tige (jbt) :** extrait aqueux provenant la base de la tige de plante céréalière, de maïs et/ou de d'autre culture obtenu par pressage selon le mode opératoire de la méthode Jubil™

**REFERENCES**

Bodilis, AM, A Bouthier, P. Castillon, F Laurent et P. Desvignes. 1998. Pilotage de la fertilisation azotée avec Jubil. Perspectives Agricoles no. 236, 1 juin 1998 (pp. 57-63). XP002691999.

Borel. C et T Simoneau. 2002. Is the ABA concentration in the sap collected by pressurizing leaves relevant for analysing drought effect on stomata? Evidence form ABA-fed leaves of transgenic plants with modified capacities to synthesize ABA. J. Exp. Bot. 53 : 287-296.

Carranca, C, A Oliveira, E Pampulha et M Odete. 2009. Temporal dynamics of soil nitrogen, carbon and microbial activity in conservative and disturbed fields amended with mature white lupine and oat residues. Geoderma 151 : 50-59

Chitrakar. R. 2010. Environmental regulation of stomate-based defense against bacterial infection in arabidopsis. Master of science in biology, University of Texas at Arlington

Chitrakar, R., Melotto, M. 2010. Assessing Stomatal Response to Live Bacterial Cells using Whole Leaf Imaging. J. Vis. Exp. (44), e2185, DOI: 10.3791/2185 (2010).

Crawford, NM. 2006. Mechanisms for nitric oxide synthesis in plants. J. Exp. Bot. 57 : 471-478

Delmotte, N, C. Knief, S. Chaffron, G. Innerebner, B. Roschitzki, R. Schlapbach, C. von Mering et JA. Vorholt. 2009. Community proteogenomics reveals insights into the physiology of phyllosphere bacteria. PNAS 106: 16428-433

Desikan, R., R. Griffiths, J. Hancock et S. Neill. 2002. A new role for an old enzyme: Nitrate reductasemediated nitric oxide generation is required for abscisic acid-induced stomatal closure in Arabidopsis thaliana. PNAS 99: 16314-16318

Desikan, R., Man-Kim Cheung, Jo Bright, Dan Henson, John T. Hancock and Steven J. Neill. 2003. ABA, hydrogen peroxide and nitric oxide signalling in stomatal guard cells. J. Exp. Bot., Vol. 55, No. 395

Dewar, RC. 2002. The Ball-Berry-Leuning and Tardieu-Davies stomatal models : synthesis and extension within a spatially aggregated picture of guard cell function. Plant Cell Environ. 25: 1383-1398

Dordas CA et C. Sioulas. 2008. Safflower yield, chlorophyll content, photosynthesis, and water use efficiency response to nitrogen fertilization under rainfed conditions. Industrial crops and products 27: 75-85

Evans, JR. 1982. Nitrogen and Photosynthesis in the Flag Leaf of Wheat (Triticum aestivum L.) PlantPhysiol. 72 : 297-302

Fischer, RA. 1968. Stomatal Opening in Isolated Epidermal Strips of Vicia faba. I. Response to Light and to CO2-free Air. Plant Physiol. 43, 1947-1952

Fuentes, JP, Markus Flury , David R. Huggins, David F. Bezdicek. 2003. Soil water and nitrogen dynamics in dryland cropping systems of Washington State, USASoil & Tillage Research 71 (2003) 33-47

Gastal, F et G. Lemaire. 2002. N uptake and distribution in crops: an agronomical and ecophysiological perspective. J. Exp. Bot. 53, No. 370, Inorganic Nitrogen Assimilation Special Issue, pp. 789-799, April 2002

Gudesblat, GE, PS Torres et AA Vojnov. 2009. Xanthomonas campestris overcomes Arabidopsis stomatal innate immunity through a DSF cell-to-cell signal regulated virulence factor. Plant Physiol. 149 : 1017-1027

HIRANO, SS. et CD. UPPER. 2000. Bacteria in the Leaf Ecosystem with Emphasis on Pseudomonas syringae-a Pathogen, Ice Nucleus, and Epiphyte. MMBR. 64 : 624-653

Huang, R. 2010. Nitrogen Assimilation-Metabolism in Relation to Potassium Use in Cauliflower (Brassica oleracea var. botrytis). (M.Sc., Dalhousie University, Halifax, Nova Scotia, Canada, 2010)

Jeuffroy, MH, B. Ney et A. Ourry. 2002. Integrated physiological and agronomic modelling of N capture and use within the plant. Journal of Experimental Botany, Vol. 53, No. 370, Inorganic Nitrogen Assimilation Special Issue, pp. 809-823, April 2002

Justes,.E, JM Meynard, B Mary et D Plénet. 1997. Diagnosis using stem base extract : Jubil Method. In Dr. Gilles Lemaire : Diagnosis of the nitrogen status in crops. 1ier janvier 1997, Springer (Berlin, Heidlebert) pp. 163-187. XP002692000

Kaiser, WM et SC. Huber. 1994. Posttranslational Regulation of Nitrate Reductase in Higher Plants. Plant Physiol. 106: 817-821

Kaiser, WM et Elke Brendle-Behnisch. 1991. Rapid Modulation of Spinach Leaf Nitrate Reductase Activity by Photosynthesis : I. Modulation in Vivo by CO2 Availability. Plant Physiol. 96, 363-367

Kaiser, WM. et Dirk Spill. 1991. Rapid Modulation of Spinach Leaf Nitrate Reductase by Photosynthesis : II. In Vitro Modulation by ATP and AMP. Plant Physiol. 96, 368-375

Laurent, F. 1998. Pilotage de la fertilisation azotée. Perspective Agricoles no. 238, 1 septembre 1998 (pp. 19-17). XP002691998.

Leveau, JHJ et Steven E. Lindow. 2001. Appetite of an epiphyte: Quantitative monitoring of bacterial sugar consumption in the phyllosphere. 3446-3453 PNAS vol. 98 u no. 6

Lindow, SE et MT. Brandl. 2003. Microbiology of the Phyllosphere. Appl. Environ. Microbiol. 69:1875-1883

Melotto, M., W. Underwood, Jessica Koczan, Kinya Nomura, and Sheng Yang He. 2006. Plant Stomata Function in Innate Immunity against Bacterial Invasion. Cell 126, 969-980

MILLER, WG., MT. BRANDL, B. QUINONES et SE. LINDOW. 2001. Biological Sensor for Sucrose Availability: Relative Sensitivities of Various Reporter Genes. Appl. Environ. Microbiol. 67: 1308-17

Mohr, TJ, Haijie Liu, Shuangchun Yan, Cindy E. Morris, José A. Castillo, Joanna Jelenska, and Boris A. Vinatzer. 2008. Naturally Occurring Nonpathogenic Isolates of the Plant Pathogen Pseudomonas syringae Lack a Type III Secretion System and Effector Gene Orthologues. J. Bacteriol., Apr. 2008, p. 2858-2870 vol. 190, No. 8

Müller, J., Eschenröder, A. 2012. Manual LEAFC3- N : Model of the Combined CO2,  , Water Vapour, and Energy Exchange Including Effects of Leaf Nitrogen content for C3 Plant Leaves (version: 2012- 08- 29) . , Martin- Luther- University of Halle- Wittenberg, Institute of Agricultural and Nutritional Sciences (Halle/ Saale, Germany) .

Müller, J, P Wernecke et W Diepenbrock. 2005. LEAFC3-N : a nitrogen sensitive extension of the CO2 and H2O gas exchange model LEAFC-3 parameterised and tested for winter wheat. Ecol. Model. 183:183-210.

Müller, J, H Braune et W Diepenbrock. 2008. Photosynthesis stomatal conductance model LEAFC3-N : spécification for barley, gerieralised nitrogen relations, and aspects of model application. Functional Plant Biol. 35:797-810.

Neill S., R.Barros, J. Bright, R. Desikan, J. Hancock, J. Harrison, P. Morris, D. Ribeiro et I. Wilson. 2008. Nitric oxide, stomatal closure, and abiotic stress. J. Exp. Bot. 59 : 165-176

Neill, SJ, R Desikan et JT. Hancock. 2003. Nitric oxide signalling in plants. New Phytologist 159 : 11-35

Oliveira, HC, EE. Saviani, JFP. Oliveira et I. Salgado. 2010. Nitrate reductase-dependent nitric oxide synthesis in the defense response of Arabidopsis thaliana against Pseudomonas syringae. Tropic Plant Pathot. 35 : 104-107

RADIN, JW et LL. PARKER. 1979. Water Relations of Cotton Plants under Nitrogen Deficiency II. environmental interactions on stomata. Plant Physiol. 64, 499-501

SHANER, DL et JS. BOYER. 1976b. Nitrate Reductase Activity in Maize (Zea mays L.) Leaves I. regulation by nitrate flux. Plant Physiol. 58, 499-504

Squire, GR et TA Mansfield. 1972. A simple method of isolating stomata on detached epidermis by low pH treatment : observations of the importance of the subsidiary cells. New Physiol. 71 : 1033-1043.

Trejo, CL, WJ. Davies et L del Mar P. Ruiz. 1993. Sensitivity of Stomata to Abscisic Acid : An Effect of the Mesophyll. Plant Physiol. (1993) 102: 497-502

Underwood, W, M Melotto et Sheng Yang He. 2007. Microreview : Role of plant stomata in bacterial invasion. Cellular Microbiology 9(7), 1621-1629

Wilkinson, S., Mark A. Bacon and William J. Davies. 2007. Nitrate signalling to stomata and growing leaves: interactions with soil drying, ABA, and xylem sap pH in maize. J. Exp. Bot., 58, No. 7, pp. 1705-1716

Wilkinson, S. et WJ. Davies. 2008. Manipulation of the apoplastic pH of intact plants mimics stomatal and growth responses to water availability and microclimatic variation. J. Exp. Bot., Advance Access published February 13, 2008 ; doi:10.1093/jxb/erm338

Yamasaki, H. 2000. Nitrite dependent nitric oxide production pathway: implications for involvement of active nitrogen species in photo-inhibition in vivo. Phii. Trans. R. Soc. Lond. B 355 : 1477-1488

Zhang, Wei, Sheng Yang He et Sarah M. Assmann. 2008. The plant innate immunity response in stomatal guard cells invokes G-protein-dependent ion channel regulation. Plant Journal 56, 984-996

**Revendications**

1. Utilisation d'engrais bactériens pour application foliaire contenant du potassium ($K_2O$), de l'azote (N) et des de bactéries stomatotaxiques et/ou stomatophiles, à pH compris entre 6, 8 et 7, 2, plus précisément entre 6, 9 et 7, 1 et avantageusement de 7 **caractérisée en ce que** ;

   ● les bactéries stomatotaxiques ont la faculté à se mouvoir à la surface de feuille (phylloplan) vers les cellules de gardes stomatiques (stomates),
   ● les bactéries stomatophiles ont elle la faculté à s'ancrer sur ou en proximité des cellules de gardes stomatiques (stomates),

   et qu'elle s'effectue ;

   ● au moment ou l'indice de nutrition azoté (NNI) est égale ou supérieur à 1,00, et/ou
   ● au moment au moment où la teneur en nitrate du jus de base de tiges ou du jus pétiolaire qui représente un stock d'ions nitrate présente dans la plante est compris entre au moins 101 et 150%, plus particulièrement entré au moins 110 et 125%, voire plus avantageusement aux environs de 120% de la valeur seuil de déclenchement d'une apport complémentaire d'engrais azoté proposée, et/ou
   ● au moment où la teneur en nitrate de la sève xylèmique ascendante est comprise entre des valeurs d'au moins 6 et 14 moles/L, plus particulièrement entre des valeurs d'au moins 8 et 12 moles/L, voire avantageusement aux environs de 10 moles/L.

2. Utilisation d'engrais bactériens pour application foliaire contenant du potassium ($K_2O$), de l'azote (N) et des de bactéries stomatotaxiques et/ou stomatophiles selon la première revendication **caractérisée en ce que** l'engrais bactérien est appliqué sur le feuillage de la canopée de cultures, plus particulièrement agronomiques, et avantageusement des grandes cultures d'hivers choisies parmi un group comprenant les céréales d'hivers telles que le blé et l'orge, ou encore le colza.

**3.** Utilisation d'engrais bactériens pour application foliaire contenant du potassium ($K_2O$), de l'azote (N) et des de bactéries stomatotaxiques et/ou stomatophiles selon une quelconque des revendications précédentes **caractérisée en ce que** l'engrais bactérien est appliqué sur le feuillage de la canopée entre ou aux stades BBCH 33 et 65, plus particulièrement BBCH 35 et 61 et avantageusement BBCH 39 et 59.

**4.** Utilisation d'engrais bactériens pour application foliaire contenant du potassium ($K_2O$), de l'azote (N) et des de bactéries stomatotaxiques et/ou stomatophiles selon la revendication précédente **caractérisée en ce que** le feuillage de la canopée est celui d'une culture céréalière d'hiver ayant produite entre 5 et 12 tonnes de matières sèches des parties aériennes (MSPA) par hectare entre ou à la mi - montaison et le début de la floraison.

**5.** Utilisation d'engrais bactériens pour application foliaire contenant du potassium ($K_2O$), de l'azote (N) et des de bactéries stomatotaxiques et/ou stomatophiles selon une quelconque des revendications précédentes **caractérisée en ce que** le potassium ainsi apporté au feuillage de la canopée représente entre 5 et 20%, et plus avantageusement environ 10% du potassium mobilisé par ce feuillage au moment de l'application dudit potassium.

**6.** Utilisation d'engrais bactériens pour application foliaire contenant du potassium ($K_2O$), de l'azote (N) et des de bactéries stomatotaxiques et/ou stomatophiles selon une quelconque des revendications précédentes **caractérisée en ce que** le ratio des teneurs en N et en K de l'engrais est compris entre 0,10 et 3,00.

**7.** Utilisation d'engrais bactériens pour application foliaire contenant du potassium ($K_2O$), de l'azote (N) et des de bactéries stomatotaxiques et/ou stomatophiles selon la revendication précédente **caractérisée en ce que** le ratio des teneurs en N et en K de l'engrais est soit ;

● faible, soit de l'ordre de 0,10 et 1,00, si NNI important est proche mais inférieur à un maximum physiologique de l'ordre 1,25 à 1,40, soit
● fort, soit de l'ordre de 1 à 3, si NNI est proche mais néanmoins supérieur à 1,00.

**8.** Utilisation d'engrais bactériens pour application foliaire contenant du potassium ($K_2O$), de l'azote (N) et des de bactéries stomatotaxiques et/ou stomatophiles selon une quelconque des revendications précédentes **caractérisée en ce que** la dose d'engrais azotés calculée et préalablement appliquée au sol, dite dose "X", représente au moins 101 et 125%, plus particulièrement au moins 105 et 115%, voire plus avantageusement aux environs de 110% de la dose X (kg-N/ha) autrement préconisée.

**9.** Utilisation d'engrais bactériens pour application foliaire contenant du potassium ($K_2O$), de l'azote (N) et des de bactéries stomatotaxiques et/ou stomatophiles selon la revendication 8 **caractérisée en ce que** la somme des fournitures du sol en azote minéral (Nm ; kg-N/ha) plus les apports d'azote fertilisant au sol impliquée dans le calcul de la dose d'engrais azotés, dite dose "X", autrement préconisée est comprise entre au moins 101 et 133%, plus particulièrement entre au moins 110 et 125%, voire plus avantageusement aux environs de 112% de 250 kg-Nm/ha pour la culture des céréales à pailles et de 320 kg-Nm/ha pour la culture du maïs-grain.

**10.** Utilisation d'engrais bactériens pour application foliaire contenant du potassium ($K_2O$), de l'azote (N) et des de bactéries stomatotaxiques et/ou stomatophiles selon la revendication 8 **caractérisée en ce que** la teneur ponctuelle des reliquats d'azote minéral des sols au moment de la récolte et impliquée dans le calcul de la dose d'engrais azotés, dite dose "X", est de l'ordre d'au moins 20 à 50 mg-N / kg-sol sec.

(a)

(b)

Figure 1

Figure 2

data Huang 2010 (Figure 3,2)

$y = -0,04x^2 + 14,47x + 2631,83$
$R^2 = 0,84$

$y = 7,11x + 2033,00$
$R^2 = 0,92$

$y = 2,6327x + 3446,5$

$y = 5,7873x + 2252$

Figure 3

data Huang 2010 (Figure 3,6)

progression de la MSPA (g/plant) — progression des taux de fertilisation N (kg/ha)

□ K0
□ K25
Ø K50

de 0 à 55 / de 55 à 220

data Huang 2010 (Figure 3,6)

progression de la MSPA (g/plant) — progression du taux de fertilisation K2O (kg/ha)

□ N0
□ ,,, N220

de 0 à 25 / de 25 à 50

**Figure 4**

Figure 5

**Figure 6**

Figure 7

Figure 8

Figure 9

Figure 10

Figuree 11

Figure 12

**Figure 13**

BTH

Tournesol

**Figure 14**

Europäisches
Patentamt

European
Patent Office

Office européen
des brevets

**RAPPORT DE RECHERCHE EUROPEENNE**

Numéro de la demande

EP 13 36 6001

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | F. LAURENT: "Pilotage de la fertilisation azotée", PERSEPCTIVES AGRICOLES, no. 238, 1 septembre 1998 (1998-09-01), pages 16-17, XP002691998, * le document en entier * | 1-8 | INV.<br>C05D1/00<br>C12N11/00 |
| A | A.-M. BODILIS, A. BOUTHIER, P. CASTILLON, F. LAURENT, P. DESVIGNES: "Pilotage de la fertilisation azotée avec Jubil", PERSEPCTIVES AGRICOLES, no. 236, 1 juin 1998 (1998-06-01), pages 57-63, XP002691999, * le document en entier * | 1-8 | |
| A | E. Justes,J. M. Meynard,B. Mary,D. Plénet: "Diagnosis Using Stem Base Extract: JUBIL Method"; "III" In: Dr. Gilles Lemaire: "Diagnosis of the Nitrogen Status in Crops", 1 janvier 1997 (1997-01-01), Springer Berlin Heidelberg, XP002692000, ISBN: 978-3-642-64506-8 pages 163-187, DOI: 10.1007/978-3-642-60684-7_10, * page 176, alinéa 10.3.4 * | 1-8 | DOMAINES TECHNIQUES RECHERCHES (IPC)<br><br>C05D<br>C12N<br>C05F<br>C05C |
| A | EP 1 457 473 A1 (SEREBRYAKOV ALEXANDR IVANOVICH [RU]; DUKHANIN VLADIMIR FEDOROVICH [RU]) 15 septembre 2004 (2004-09-15) * alinéa [0028] * * alinéa [0005] - alinéa [0009] * | 1-8 | |

Le présent rapport a été établi pour toutes les revendications

| Lieu de la recherche | Date d'achèvement de la recherche | Examinateur |
|---|---|---|
| La Haye | 1 juillet 2013 | Cardin, Aurélie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 13 36 6001

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

01-07-2013

| Document brevet cité au rapport de recherche | | Date de publication | Membre(s) de la famille de brevet(s) | | Date de publication |
|---|---|---|---|---|---|
| EP 1457473 | A1 | 15-09-2004 | CA | 2464915 A1 | 08-05-2003 |
| | | | EP | 1457473 A1 | 15-09-2004 |
| | | | LT | 2004056 A | 25-10-2004 |
| | | | RU | 2203869 C1 | 10-05-2003 |
| | | | UA | 76234 C2 | 15-09-2004 |
| | | | US | 2005115291 A1 | 02-06-2005 |
| | | | WO | 03037828 A1 | 08-05-2003 |

EPO FORM P0460

Pour tout renseignement concernant cette annexe : voir Journal Officiel de l'Office européen des brevets, No.12/82

# EP 2 647 612 A1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BODILIS, AM ; A BOUTHIER ; P. CASTILLON ; F LAURENT ; P. DESVIGNES.** Pilotage de la fertilisation azotée avec Jubil. *Perspectives Agricoles,* 01 Juin 1998, 57-63
- **BOREL. C ; T SIMONEAU.** Is the ABA concentration in the sap collected by pressurizing leaves relevant for analysing drought effect on stomata? Evidence form ABA-fed leaves of transgenic plants with modified capacities to synthesize ABA. *J. Exp. Bot.,* 2002, vol. 53, 287-296
- **CARRANCA, C ; A OLIVEIRA ; E PAMPULHA ; M ODETE.** Temporal dynamics of soil nitrogen, carbon and microbial activity in conservative and disturbed fields amended with mature white lupine and oat residues. *Geoderma,* 2009, vol. 151, 50-59
- **CHITRAKAR. R.** Environmental regulation of stomate-based defense against bacterial infection in arabidopsis. *Master of science in biology,* 2010
- **CHITRAKAR, R. ; MELOTTO, M.** Assessing Stomatal Response to Live Bacterial Cells using Whole Leaf Imaging. *J. Vis. Exp.,* 2010, e2185
- **CRAWFORD, NM.** Mechanisms for nitric oxide synthesis in plants. *J. Exp. Bot.,* 2006, vol. 57, 471-478
- **DELMOTTE, N ; C. KNIEF ; S. CHAFFRON ; G. INNEREBNER ; B. ROSCHITZKI ; R. SCHLAPBACH ; C. VON MERING ; JA. VORHOLT.** Community proteogenomics reveals insights into the physiology of phyllosphere bacteria. *PNAS,* 2009, vol. 106, 16428-433
- **DESIKAN, R. ; R. GRIFFITHS ; J. HANCOCK ; S. NEILL.** A new role for an old enzyme: Nitrate reductasemediated nitric oxide generation is required for abscisic acid-induced stomatal closure in Arabidopsis thaliana. *PNAS,* 2002, vol. 99, 16314-16318
- **DESIKAN, R. ; MAN-KIM CHEUNG ; JO BRIGHT ; DAN HENSON ; JOHN T. HANCOCK ; STEVEN J. NEILL.** ABA, hydrogen peroxide and nitric oxide signalling in stomatal guard cells. *J. Exp. Bot.,* 2003, vol. 55 (395
- **DEWAR, RC.** The Ball-Berry-Leuning and Tardieu-Davies stomatal models : synthesis and extension within a spatially aggregated picture of guard cell function. *Plant Cell Environ.,* 2002, vol. 25, 1383-1398
- **DORDAS CA ; C. SIOULAS.** Safflower yield, chlorophyll content, photosynthesis, and water use efficiency response to nitrogen fertilization under rainfed conditions. *Industrial crops and products,* 2008, vol. 27, 75-85
- **EVANS, JR.** Nitrogen and Photosynthesis in the Flag Leaf of Wheat (Triticum aestivum L. *PlantPhysiol.,* 1982, vol. 72, 297-302
- **FISCHER, RA.** Stomatal Opening in Isolated Epidermal Strips of Vicia faba. I. Response to Light and to CO2-free Air. *Plant Physiol.,* 1968, vol. 43, 1947-1952
- **FUENTES, JP ; MARKUS FLURY ; DAVID R. HUGGINS ; DAVID F. BEZDICEK.** Soil water and nitrogen dynamics in dryland cropping systems of Washington State. *USASoil & Tillage Research,* 2003, vol. 71, 33-47
- **GASTAL, F ; G. LEMAIRE.** N uptake and distribution in crops: an agronomical and ecophysiological perspective. *J. Exp. Bot.,* Avril 2002, vol. 53 (370), 789-799
- **GUDESBLAT, GE ; PS TORRES ; AA VOJNOV.** Xanthomonas campestris overcomes Arabidopsis stomatal innate immunity through a DSF cell-to-cell signal regulated virulence factor. *Plant Physiol.,* 2009, vol. 149, 1017-1027
- **HIRANO, SS. ; CD. UPPER.** Bacteria in the Leaf Ecosystem with Emphasis on Pseudomonas syringae-a Pathogen. *Ice Nucleus, and Epiphyte. MMBR,* 2000, vol. 64, 624-653
- **HUANG, R.** Nitrogen Assimilation-Metabolism in Relation to Potassium Use in Cauliflower (Brassica oleracea var. botrytis. *M.Sc.,* 2010
- **JEUFFROY, MH ; B. NEY ; A. OURRY.** Integrated physiological and agronomic modelling of N capture and use within the plant. *Journal of Experimental Botany,* Avril 2002, vol. 53 (370), 809-823
- Diagnosis using stem base extract : Jubil Method. **JUSTES,.E ; JM MEYNARD ; B MARY ; D PLÉNET.** Dr. Gilles Lemaire : Diagnosis of the nitrogen status in crops. Springer, 1997, 163-187
- **KAISER, WM ; SC. HUBER.** Posttranslational Regulation of Nitrate Reductase in Higher Plants. *Plant Physiol.,* 1994, vol. 106, 817-821
- **KAISER, WM ; ELKE BRENDLE-BEHNISCH.** Rapid Modulation of Spinach Leaf Nitrate Reductase Activity by Photosynthesis : I. Modulation in Vivo by CO2 Availability. *Plant Physiol.,* 1991, vol. 96, 363-367

- **KAISER, WM. ; DIRK SPILL.** Rapid Modulation of Spinach Leaf Nitrate Reductase by Photosynthesis : II. In Vitro Modulation by ATP and AMP. *Plant Physiol.,* 1991, vol. 96, 368-375
- **LAURENT, F.** Pilotage de la fertilisation azotée. *Perspective Agricoles,* 01 Septembre 1998, 19-17
- **LEVEAU, JHJ ; STEVEN E. LINDOW.** Appetite of an epiphyte: Quantitative monitoring of bacterial sugar consumption in the phyllosphere. *PNAS,* 2001, vol. 98 (6), 3446-3453
- **LINDOW, SE ; MT. BRANDL.** Microbiology of the Phyllosphere. *Appl. Environ. Microbiol.,* 2003, vol. 69, 1875-1883
- **MELOTTO, M. ; W. UNDERWOOD ; JESSICA KOCZAN ; KINYA NOMURA ; SHENG YANG HE.** Plant Stomata Function in Innate Immunity against Bacterial Invasion. *Cell,* 2006, vol. 126, 969-980
- **MILLER, WG. ; MT. BRANDL ; B. QUINONES ; SE. LINDOW.** Biological Sensor for Sucrose Availability: Relative Sensitivities of Various Reporter Genes. *Appl. Environ. Microbiol.,* 2001, vol. 67, 1308-17
- **MOHR, TJ ; HAIJIE LIU ; SHUANGCHUN YAN ; CINDY E. MORRIS ; JOSÉ A. CASTILLO ; JOANNA JELENSKA ; BORIS A. VINATZER.** Naturally Occurring Nonpathogenic Isolates of the Plant Pathogen Pseudomonas syringae Lack a Type III Secretion System and Effector Gene Orthologues. *J. Bacteriol.,* Avril 2008, vol. 190 (8), 2858-2870
- **MÜLLER, J. ; ESCHENRÖDER, A.** Manual LEAFC3-N : Model of the Combined CO2. *Water Vapour, and Energy Exchange Including Effects of Leaf Nitrogen content for C3 Plant Leaves,* 2012
- **MÜLLER, J ; P WERNECKE ; W DIEPENBROCK.** LEAFC3-N : a nitrogen sensitive extension of the CO2 and H2O gas exchange model LEAFC-3 parameterised and tested for winter wheat. *Ecol. Model.,* 2005, vol. 183, 183-210
- **MÜLLER, J ; H BRAUNE ; W DIEPENBROCK.** Photosynthesis stomatal conductance model LEAFC3-N : spécification for barley, gerieralised nitrogen relations, and aspects of model application. *Functional Plant Biol,* 2008, vol. 35, 797-810
- **NEILL S. ; R.BARROS ; J. BRIGHT ; R. DESIKAN ; J. HANCOCK ; J. HARRISON ; P. MORRIS ; D. RIBEIRO ; I. WILSON.** Nitric oxide, stomatal closure, and abiotic stress. *J. Exp. Bot.,* 2008, vol. 59, 165-176
- **NEILL, SJ ; R DESIKAN ; JT. HANCOCK.** Nitric oxide signalling in plants. *New Phytologist,* 2003, vol. 159, 11-35
- **OLIVEIRA, HC ; EE. SAVIANI ; JFP. OLIVEIRA ; I. SALGADO.** Nitrate reductase-dependent nitric oxide synthesis in the defense response of Arabidopsis thaliana against Pseudomonas syringae. *Tropic Plant Pathot,* 2010, vol. 35, 104-107
- **RADIN, JW ; LL. PARKER.** Water Relations of Cotton Plants under Nitrogen Deficiency II. environmental interactions on stomata. *Plant Physiol.,* 1979, vol. 64, 499-501
- **SHANER, DL ; JS. BOYER.** Nitrate Reductase Activity in Maize (Zea mays L.) Leaves I. regulation by nitrate flux. *Plant Physiol.,* 1976, vol. 58, 499-504
- **SQUIRE, GR ; TA MANSFIELD.** A simple method of isolating stomata on detached epidermis by low pH treatment : observations of the importance of the subsidiary cells. *New Physiol.,* 1972, vol. 71, 1033-1043
- **TREJO, CL ; WJ. DAVIES ; L DEL MAR P. RUIZ.** Sensitivity of Stomata to Abscisic Acid : An Effect of the Mesophyll. *Plant Physiol.,* 1993, vol. 102, 497-502
- **UNDERWOOD, W ; M MELOTTO ; SHENG YANG HE.** Microreview : Role of plant stomata in bacterial invasion. *Cellular Microbiology,* 2007, vol. 9 (7), 1621-1629
- **WILKINSON, S. ; MARK A. BACON ; WILLIAM J. DAVIES.** Nitrate signalling to stomata and growing leaves: interactions with soil drying, ABA, and xylem sap pH in maize. *J. Exp. Bot.,* 2007, vol. 58 (7), 1705-1716
- **WILKINSON, S. ; WJ. DAVIES.** Manipulation of the apoplastic pH of intact plants mimics stomatal and growth responses to water availability and microclimatic variation. *J. Exp. Bot., Advance Access,* 13 Février 2008
- **YAMASAKI, H.** Nitrite dependent nitric oxide production pathway: implications for involvement of active nitrogen species in photo-inhibition in vivo. *Phii. Trans. R. Soc. Lond. B,* 2000, vol. 355, 1477-1488
- **ZHANG, WEI ; SHENG YANG HE ; SARAH M. ASSMANN.** The plant innate immunity response in stomatal guard cells invokes G-protein-dependent ion channel regulation. *Plant Journal,* 2008, vol. 56, 984-996